# EUROPEAN PATENT SPECIFICATION

(11) **EP 0 330 781 B1**
(45) Date of publication and mention of the grant of the patent: **06.03.1996**
(21) Application number: 88310203.0
(22) Date of filing: 31.10.1988
(51) Int. Cl.: C12N 15/45, C12N 15/86, A61K 39/155

(54) **Vaccine against rinderpest virus using recombinant vaccinia virus**
Vakzin gegen Rinderpestvirus unter Benutzung von rekombinantem Vacciniavirus
Vaccin contre le virus de rinderpest utilisant le virus de vaccinia recombinant

(30) Priority: 29.02.1988 JP 44134/88
(43) Date of publication of application: 06.09.1989
(73) Proprietor: TOA NENRYO KOGYO KABUSHIKI KAISHA, Tokyo 100 (JP)
(72) Inventor: Yamanouchi, Kazuya, Fuchu-shi Tokyo (JP); Yoshikawa, Yasuhiro, Hoya-shi Tokyo (JP); Tsukiyama, Kyoko, Meguro-ku Tokyo (JP); Asano, Kaori, Asaka-shi Saitama (JP); Maruyama, Tadashi, Koganei-shi Tokyo (JP); Sugimoto, Masanobu, Shiki-shi Saitama (JP)
(74) Representative: Baverstock, Michael George Douglas

(56) References cited:
- EP-A- 305 229
- EP-A- 0 257 994
- PROC. NATL. ACAD. SCI. USA, vol. 85, February 1988, pages 1252-1256; R. DRILLIEN et al.
- BIOLOGICAL ABSTRACTS, vol. 84, no. 11, 1987, abstract no. 107714, Philadelphia, US; K. TSUKIYAMA et al.
- CHEMICAL ABSTRACTS, vol. 109, 1988, page 184, abstract no. 223508s, Columbus, Ohio, US; K. TSUKIYAMA et al.

## Description

The present invention relates to a recombinant vaccinia virus vaccine against the rinderpest virus. The present vaccine is useful in the field of livestock farming and dairy farming.

Rinderpest is prevalent in many areas of the world, such as the Middle and Near East and Africa, for example, and therefore, intensive research is underway into the development of vaccines for the rinderpest virus.

Due to the adverse conditions, including a relatively high temperature ambient atmosphere, in these areas, a vaccine having a high thermostability being able to be stored for long periods, and exhibiting a high, long term immunological effect, is strongly desired.

In the development of vaccines through genetic engineering, processes wherein a gene coding for an antigen protein of the vaccine is expressed in E. Coli, yeast or animal cells to obtain the antigen protein, and processes wherein a gene coding for an antigen protein is integrated into a vaccinia virus genome to construct a recombinant virus for the production of a live vaccine, are known. For example a recombinant vaccinia virus encoding the hemagglutinin or fusion protein of measles virus is described by R. Drillien et al. in Proc. Natl. Acad. Sci. 1988, Vol 85, 1252-1256. But, to carry out such processes, a gene coding for an antigen protein of the rinderpest virus must be cloned.

A rinderpest virus strain L that can be grown in Vero cells has been constructed (F. Kobune et al., Archives of Virology, 68, 271-277, 1981). Also, a rinderpest virus strain L (L 13) that can be grown in Vero cells has been developed (J. Gen. Virol. 67, 271, 1986).

The cloning of a cDNA coding for an F protein of the canine distemper virus has been disclosed by S.E.H. Russell, J. Gen. Virol. 66, 435-441, 1985.

The present inventors cloned a cDNA coding for an F protein and a cDNA coding for an H protein of the rinderpest virus, as disclosed in EP Publication No. 0257994.

A recombinant live vaccine comprising an antigen gene of the Morbillivirus family inserted into a vaccinia virus gene is described in EP-A-0305229. However the particular virus strain used is not specified.

Accordingly, the present invention provides a recombinant vaccine comprising an antigen gene of the rinderpest virus inserted into a vaccinia virus gene of vaccinia virus strain LC16m0 or LC16m8 and process for the production of an a method of use of the recombinant vaccine.

More specifically, the present invention provides a recombinant vaccinia virus wherein a gene coding for an antigenic protein of the rinderpest virus and a viral promoter which can express that gene have been inserted in a vaccinia virus gene which is not essential for the growth of the vaccinia virus LC16m0 or LC16m8.

The present invention also provides a process for the production of a recombinant vaccinia virus, comprising the steps of
(1) preparing a plasmid comprising a gene coding for an antigenic protein of the rinderpest virus and a viral promoter which can express that gene, as well as a gene derived from the vaccinia virus but not essential for the growth of the vaccinia virus, wherein the gene coding for the antigenic protein and the viral promoter have been inserted in the gene not essential for the growth of the vaccinia virus LC16m0 or LC16m8;
(2) subjecting the plasmid to a recombination with a vaccinia virus LC16m0 or LC16m8 and
(3) selecting a recombinant vaccinia virus wherein a gene corresponding to the gene not essential for the growth of the vaccinia virus has been inactivated.

Moreover, the present invention provides a vaccine against the rinderpest virus comprising a recombinant vaccinia virus wherein a gene coding for an antigenic protein of the rinderpest virus and a viral promoter which can express that gene have been inserted in a gene which is not essential for the growth of the vaccinia virus LC16m0 or LC16m8.

The vaccine of the present invention can be used, to immunize by vaccination, a mammal, against the rinderpest virus, by injecting the mammal with the above-mentioned recombinant vaccinia virus or the above-mentioned vaccine.

Finally the present invention also provides a plasmid comprising a gene coding for an antigenic protein of the rinderpest virus and a viral promoter which can express that gene, as well as a gene derived from the vaccinia virus but not essential for the growth of the vaccinia virus, wherein the gene coding for the antigenic protein and the viral promoter have been inserted in the gene not essential for the growth of the vaccinia virus characterised in that:
(1) the viral promoter is vaccinia virus 7.5 K protein gene promoter (7.5 K) and the vaccinia virus gene not essential for the growth of vaccinia virus is a vaccinia virus hemagglutinin gene (H); or
(2) the viral promoter is poxvirus A type inclusion body gene promoter (ATIp), and the vaccinia virus gene not essential for the growth of vaccinia virus is H; or
(3) the viral promoter is ATIp, and the vaccinia virus gene not essential for the growth of vaccinia virus is vaccinia virus thymidine kinase gene (TK).

The invention will now be described with reference to the examples and accompanying drawings.
Figure 1 represents a restriction enzyme cleavage map of a cDNA insert RV-H-2 in a plasmid pDH-RVH-2 and a restriction enzyme cleavage map of a cDNA insert RV-F-2 in a plasmid pDH-RVF-2, wherein the thick line in the RV-H-2 represents a portion corresponding to a nucleotide sequence coding for a hemagglutinin protein (H protein) of the rinderpest virus shown in Figs. 2-1 to 2-4, and the thick line in the RV-F-2 represents a portion corresponding to a nucleotide sequence coding for a fusion protein (F protein) of the rinderpest virus shown in Figs. 3-1 to 3-4;
Figs. 2-1 to 2-4 represent a nucleotide sequence containing a hemagglutinin gene of the rinderpest virus, wherein, in the sequence, ATG at the 21st to 23rd position is a translation start codon and TAG at the 1848th to 1850th position is a translation stop codon, and a reading frame flanked by these codons (represented by a thick line in Fig. 1, RV-H-2) shows the codes for a hemagglutinin protein;
Figs. 3-1 to 3-5 represent a nucleotide sequence containing a fusion protein gene of the rinderpest virus, wherein, in the sequence, ATG at the 587th to 589th position is a translation start codon and TAG at the 2225th to 2227th position is a translation stop codon, and a reading frame flanked by these codons (represented by a thick line in Fig. 1, RV-F-2) shows the codes for a fusion protein;
Fig. 4 shows a construction process of the plasmid pHA13 containing a hemagglutinin gene;
Fig. 5 shows a structure of the hemagglutinin gene of the vaccinia virus;
Fig. 6 shows a construction process of the plasmid pHA-7.5pN-RVH containing a 7.5K promoter followed by the rinderpest virus hemagglutinin gene (RVH) inserted into the vaccinia virus hemagglutinin gene (HA);
Fig. 7 shows a construction process of the plasmid p18-ATI-pro536 containing an ATI promoter;
Fig. 8 shows a construction process of the plasmid pA2-9 containing an ATI promoter;
Fig. 9 shows a construction process of the plasmid pHAA2-9 containing an ATI promoter inserted into the vaccinia virus hemagglutinin gene (HA);
Fig. 10 shows a construction process of the plasmid pHA-ATIp-RVH containing an ATI promoter followed by the rinderpest virus hemagglutinin gene (RVH) inserted into a vaccinia virus hemagglutinin gene (HA);
Fig. 11 shows a nucleotide sequence of a synthesized 5′-portion of the rinderpest virus hemagglutinin gene (RVH) having a modified 5′-non-translation region;
Fig. 12 shows a construction process of the plasmid p19-RVH3 containing an RVH gene by ligation of the synthetic RVH gene shown in Fig. 11 and a cDNA of the RVH gene. The recombinant RVH gene is designated as RVH3;
Fig. 13 shows a construction process of the plasmid pHA-7.5pN-RVH3 containing a 7.5pN promoter followed by the RVH3 inserted into an HA gene, wherein the 7.5pN-RVH3 has the same orientation as the HA gene, and the plasmid pHA-ATIp-RVH3 containing the plasmid ATIp followed by the RVH3 inserted into a HA gene, wherein the ATIp-RVH3 has an orientation opposite to that of the HA gene;
Fig. 14 shows a cloning process of a TK gene of the vaccinia virus WR;
Fig. 15 shows a construction process of the plasmid pTK-7.5pN containing a 7.5pN promoter inserted into a TK gene;
Fig. 16 shows a construction process of the plasmid pUC119-RVF3 containing the rinderpest virus fusion protein gene (RVF3);
Fig. 17 shows a construction process of the plasmid pTK-7.5pN-RVF3 containing the 7.5pN plasmid followed by the RVF3 inserted into a TK gene;
Fig. 18 shows an electrophoresis pattern confirming the production of the rinderpest virus hemagglutinin (RVH) by immunoprecipitation;
Fig. 19 is a graph representing a neutralizing antibody titer in sera from rabbits vaccinated with different viruses including recombinant viruses of the present inventions, mo-ATIp-RVH and mo-7.5pN-RVH, as well as a non-recombinant control virus mo;
Fig. 20 is a graph representing changes in the body temperature of rabbits vaccinated with a recombinant virus of the present invention or non-vaccinated control rabbits, and then exposed to the rinderpest virus;
Fig. 21 shows an electrophoresis pattern representing the formation of an anti-rinderpest virus antibody in rabbits vaccinated with a virus of the present invention or non-recombinant control virus mO, and in an anti-rinderpest virus serum; and
Fig. 22 shows an electrophoresis pattern confirming the production of the rinderpest virus hemagglutinin (RVH3 and others) by immunoprecipitation.

To construct a recombinant vaccinia virus effective as a live vaccine by inserting an antigen gene of the rinderpest virus into a vaccinia virus, the antigen gene of rinderpest virus must be inserted into a gene region which is not essential for the growth of the vaccinia virus. This insertion can be conveniently carried out through a homologous recombination. Therefore, in the present invention, a plasmid is constructed wherein a gene coding for an antigen protein of the rinderpest virus and a promoter which can direct the expression of that gene, are both inserted into a gene region which is not essential for the growth of vaccinia virus. Next, the plasmid is used to insert the gene coding for the antigen protein of the rinderpest virus and the promoter for the expression of the gene into the gene region not essential for the growth of vaccinia virus, through a homologous recombination between the plasmid and the vaccinia virus gene.

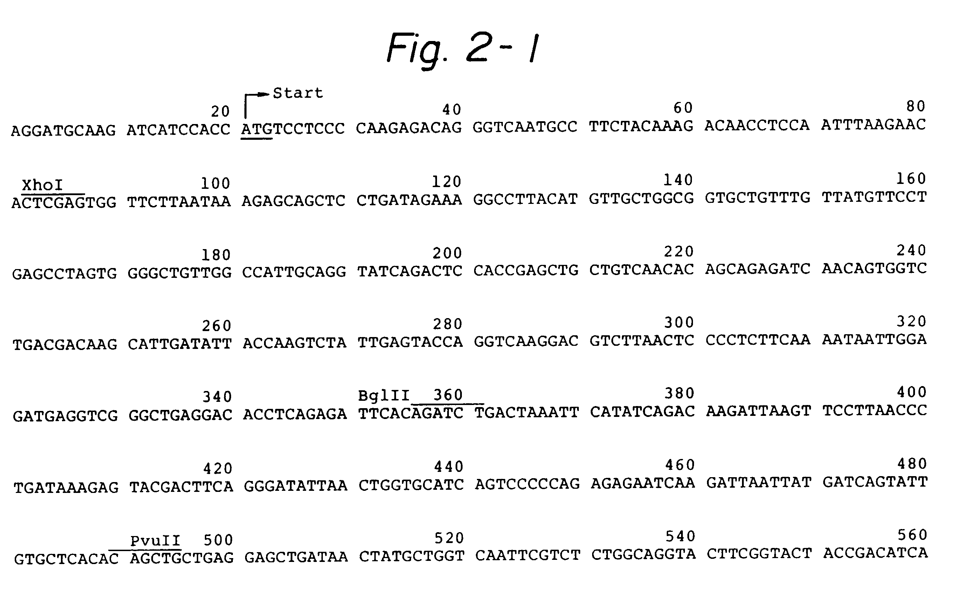

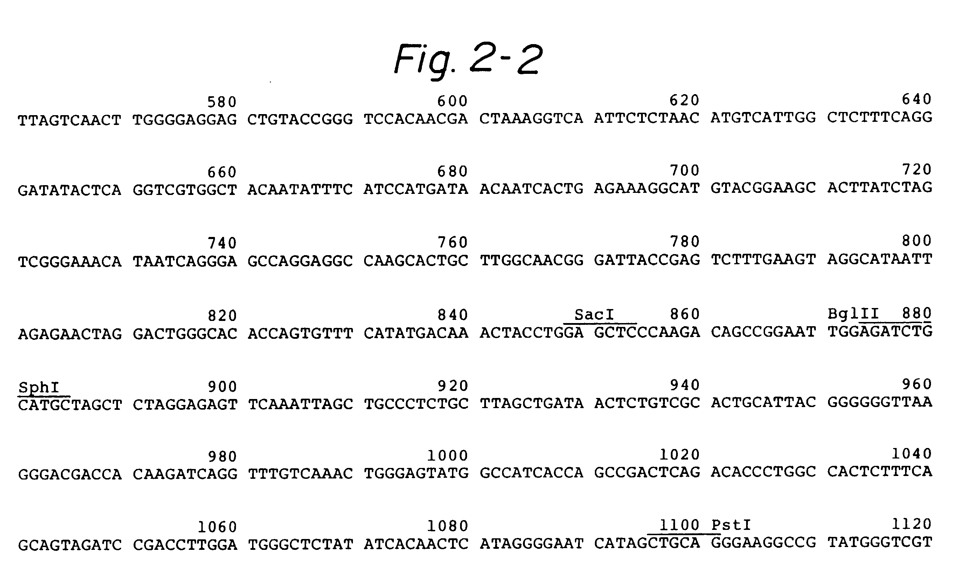

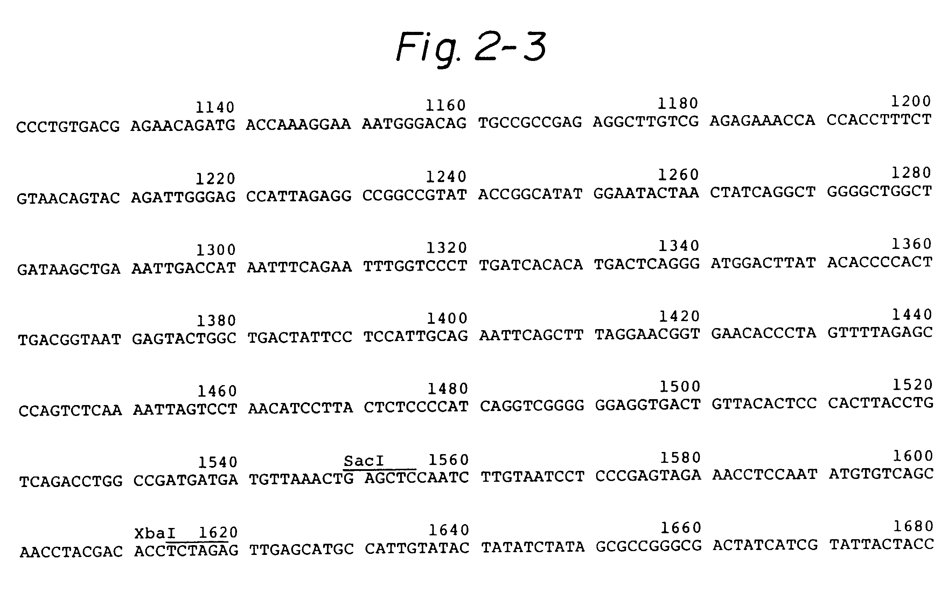

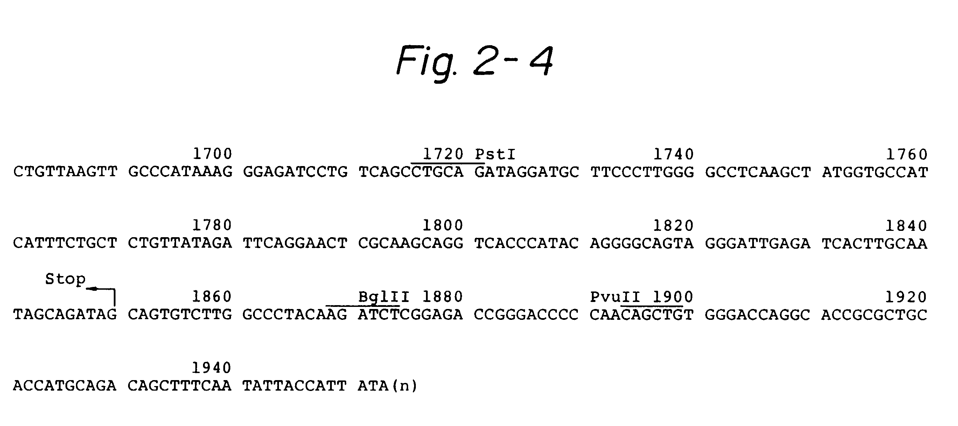

Since it is considered that the proteins effective as an antigen of the rinderpest virus are a hemagglutinin protein (RVH) and a fusion protein (RVF), according to the present invention, a gene coding for the RVH or RVF is used as the antigen gene. These proteins may be full length, or be a part thereof to the extent that the antigenic property is maintained. A procedure for cloning the genes coding for these antigenic proteins is disclosed in Example 1. Further, the nucleotide sequences of these genes are shown in Figs. 2-1 to 2-4, and Figs. 3-1 to 3-4.

The promoter for the expression of the antigen gene may be any promoter which can direct the expression of a desired structural gene in the virus, and includes, for example, a promoter of an A-type inclusion body gene (ATI) of the cowpox virus, a promoter of a 7.5K protein gene of the vaccinia virus, and the like. A procedure for cloning the ATI promoter is disclosed in Example 4. The 7.5K promoter can be cloned and, for example, a promoter cloned in the plasmid p7.5-18 can be used. Further, this promoter can be chemically synthesized. These promoters may have a naturally occurring nucleotide sequence or a modified nucleotide sequence.

The gene region which is not essential for the growth of vaccinia virus is, for example, a vaccinia virus hemagglutinin gene (H), a vaccinia virus thymidine kinase gene (TK), or the like. A procedure for cloning the vaccinia virus hemagglutinin gene (H) is described in Example, 2, and a procedure for cloning the vaccinia virus thymidine kinase gene (TK) is described in Example 14.

According to the present invention, the plasmids for recombination can be constructed by combination of the above-mentioned gene elements.

In one embodiment, a plasmid is used wherein a rinderpest virus hemagglutinin structural gene (RVH) and a 7.5K promoter have been inserted into a vaccinia virus hemagglutinin gene. These plasmids are, for example, pHA-7.5pN-RVH and pHA-7.5pN-RVH3. As a representative of microorganisms which contain these plasmids, Escherichia coli MV-GA-7.5pN-RVH, which contains pHA-7.5pN-RVH, was deposited with Fermentation Research Institute Agency of Industrial Science and Technology (FRI), 1-3 Higashi 1-chome Tsukuba-shi Ibaraki-ken Japan, as FERM BP-1765, under the Budapest treaty, on February 25, 1988.

In another embodiment, a plasmid is used wherein the rinderpest virus hemagglutinin structural gene (RVH) and the cowpox virus A-type inclusion body gene promoter (ATIp) have been inserted into a vaccinia virus hemagglutinin gene (H). These plasmids are, for example, pHA-ATIp-RVH and pHA-ATIp-RVH3. As a representative of microorganisms containing these plasmids, Escherichia coli MV-HA-ATIp-RVH, which contains pHA-ATIp-RVH, was deposited with the FRI as FERM BP-1767 under the Budapest treaty on February 25, 1988.

In another embodiment, a plasmid is used wherein the rinderpest virus fusion protein structural gene (RVF) and the 7.5K protein gene promoter have been inserted into a vaccinia virus thymidine kinase gene (TK). Such a plasmid is, for example, the plasmid pTK-7.5pN-RVF. Escherichia coli DH-TK-7.5pN-RVF, which contains pTK-7.5pN-RVF, was deposited with the FRI as FERM BP-1766 under the Budapest treaty on February 25, 1988.

Further, the present invention provides, for example, a plasmid wherein the rinderpest virus hemagglutinin structural gene (RVH) and the cowpox virus A-type inclusion body gene promoter (ATIp) have been inserted into a vaccinia virus thymidine kinase gene (TK); a plasmid wherein the rinderpest virus fusion protein structural gene (RVF) and the cowpox virus T-type inclusion body gene promoter (ATIp) have been inserted into a vaccinia virus hemagglutinin gene (H); a plasmid wherein the rinderpest virus fusion protein structural gene (RVF) and the cowpox virus T-type inclusion body gene promoter (ATPp) have been inserted into a vaccinia virus thymidine kinase gene (TK); and a plasmid wherein the rinderpest virus fusion protein structural gene (RVF) and the vaccinia virus 7.5K protein gene promoter (7.5Kp) have been inserted into a vaccinia virus hemagglutinin gene (H), are used.

The recombinant vaccinia virus of the present invention comprising a rinderpest virus antigen gene and an appropriate promoter for the expression of the antigen gene is obtained through a homologous recombination of one of the above-mentioned plasmids and the vaccinia virus LC16m0 or LC16m8 or by homologous recombination of a plasmid wherein the rinderpest virus hemagglutinin structural gene (RVH) and the vaccinia virus 7.5K protein gene promoter (7.5Kp) have been inserted into a cowpox virus thymidine kinase gene, with vaccinia virus LC16m0, or LC16m8.

For this purpose, vaccinia virus strains LC16m0 and LC16m8 were obtained, as strains having a low neurovirulence, from the parent strain LO.

These strains have the properties set forth in Table 1.

The process for the construction and the properties of these parent viruses are disclosed in detail in Rinsho To Virus, Vol 3, No. 3, 229-235, 1985 and in S. Hashizume et al., Vaccinia Viruses as vectors for vaccine antigens, published 1985 by Elsevier Science Publishing Co., Inc., pp. 87-99. These mutant viruses are available from Chiba-Serum Institute, Japan.

**Table 1**

| Virus strain | LO | LC16m0 | LC16m8 |
|---|---|---|---|
| Temperature at which virus cannot proliferate on RK cells | >41°C | 41°C | 40.5°C |
| Plaque size on RK cells | Large | Medium | Medium |
| Pock size | Large | Medium | Small |

| Proliferation potency on | | | |
|---|---|---|---|
| CAM | +++ | +++ | +++ |
| Vero cells | +++ | +++ | + |
| RK cells | +++ | +++ | +++ |
| CEF cells | ++ | ++ | ++ |

| Neurovirulence to central nervous system | | | |
|---|---|---|---|
| Proliferation | | | |
| Rabbit | ++ | + | ± |
| Monkey | +++ | + | + |
| Mouse | + | | |

| Invasion | | | |
|---|---|---|---|
| Mouse | +++ | - | - |

| Proliferation in skin | | | |
|---|---|---|---|
| Rabbit | +++ | ++ | + |
| Human | ++ | ++ | + |

| Antibody productivity | | | |
|---|---|---|---|
| Rabbit | ++ | +++ | ++ |
| Human | ++ | +++ | ++ |

If a plasmid in which the rinderpest virus antigen gene and a promoter therefor have been inserted into a vaccinia virus hemagglutinin gene is used, the antigen gene is inserted into hemagglutinin gene region of the vaccinia virus. On the other hand, if a plasmid in which the rinderpest virus antigen gene and a promoter therefor have been inserted into a vaccinia virus thymidine kinase gene is used, the antigen gene is inserted into the thymidine kinase gene region of the vaccinia virus.

The recombination can be carried out by a conventional transfection technique. For example, where the vaccinia virus hemagglutinin gene region is used as a homologous recombination region, cultured animal cells, such as Vero cells or rabbit renal cells, are infected with a vaccinia virus, and then the plasmid is transfected into the infected animal cells using the Caphosphate method, to obtain candidate viruses. Next, a monolayer of animal cells such as RK13 cells are infected with the candidate viruses to form plagues, and a virus which does not agglutinate chicken erythrocytes (HA⁻) is selected. Where a vaccinia virus thymidine kinase gene is used as the homologous recombination region, TK⁻ mutant animal cells are infected with the vaccinia virus, and then the plasmid is transfected into the infected animal cells and the transfected cells are cultured in the presence of bromodeoxyuridine (BUdR), to select the candidate recombinant viruses.

After cloning, the above-mentioned candidate virus is infected to a monolayer of RK13 cells, to form plaques. The plaques are adsorbed to a nitro-cellulose filter or nylon membrane, and after denaturation of a virus DNA from the plaques, the filter or membrane is subjected to hybridization with a rinderpest virus hemagglutinin gene fragment or rinderpest virus fusion protein gene fragment to confirm the integration of the rinderpest virus hemagglutinin gene or rinderpest virus fusion protein gene into the vaccinia virus genome. Alternatively, the incorporation of the rinderpest virus hemagglutinin gene or rinderpest virus fusion protein gene into the vaccinia virus can be tested by subjecting a lysate of animal cells infected with the vaccinia virus to be tested to immunoprecipitation, using an antibody to the rinderpest virus hemagglutinin or the rinderpest virus fusion protein.

Particular recombinant vaccinia viruses of the present invention include a recombinant vaccinia virus mO-ATIp-RVH derived from a recombination of the plasmid pHA-ATIp-RVH and the vaccinia virus mO, as well as a recombinant vaccinia virus mO-7.5pN-RVH derived from a recombination of the plasmid pHA-7.5pN-RVH and the vaccinia virus mO.

Note, embodiments of the present invention exemplify the use of the vaccinia virus mO.

It was confirmed that a rabbit injected with the above-mentioned recombinant vaccinia virus produces a neutralizing antibody against the rinderpest virus (see Example 20), and is resistant to infection of the rinderpest virus (see Example 21).

### EXAMPLES

The present invention will now be further illustrated by, but is by no means limited to, the following examples.

In the Examples, the following buffers for enzyme reaction were used.

### (1) Buffer for restriction enzyme

(a) Middle salt concentration buffer (pH7.5)

| | |
|---|---|
| NaCl | 50 mM |
| Tris-HCl | 10 mM |
| MgCl₂ | 10 mM |
| Dithiothreitol | 1 mM |

(b) High salt concentration buffer (pH7.5) 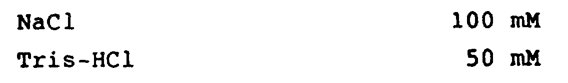 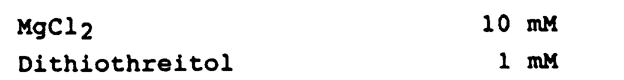
(c) SmaI buffer (pH8.0)

| | |
|---|---|
| KCl | 20 mM |
| Tris-HCl | 10 mM |
| MgCl₂ | 10 mM |
| Dithiothreitol | 1 mM |

(d) NruI buffer (pH8.0)

| | |
|---|---|
| Tris-HCl | 10 mM |
| MgCl₂ | 7 mM |
| KCl | 150 mM |
| 2-Mercaptoethanol | 7 mM |
| Bovine serum albumin | 100 µg/ml |

### (2) Ligation buffer (pH7.6)

| | |
|---|---|
| Tris-HCl | 66 mM |
| MgCl₂ | 5 mM |
| Dithiothreitol | 5 mM |
| ATP | 1 mM |

### (3) Nick-translation buffer (pH7.2)

| | |
|---|---|
| Tris-HCl | 50 mM |
| MgSO₄ | 10 mM |
| Dithiothreitol | 0.1 mM |
| Bovine serum albumin | 50 µg/ml |

### (4) Exonuclease III buffer (pH7.5)

| | |
|---|---|
| Tris-HCl | 50 mM |
| MgCl₂ | 50 mM |
| DTT | 5 mM |
| Bovine serum albumin | 50 µg/ml |

### (5) SI nuclease buffer (pH4.6)

| | |
|---|---|
| Sodium acetate | 50 mM |
| Zinc acetate | 1 mM |
| NaCl | 250 mM |
| Bovine serum albumin | 50 µg/ml |

### (6) Mung bean nuclease buffer (pH5.0)

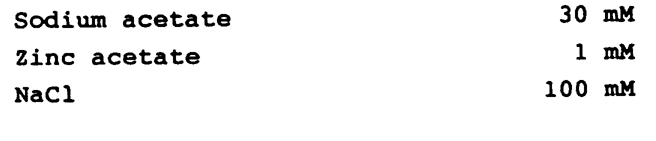

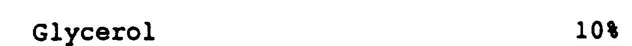

### Example 1 Cloning of rinderpest virus antigen gene

Since it is believed that the proteins effective as antigens to the rinderpest virus are the hemagglutinin protein (H protein) and the fusion protein (F protein), according to the present invention, the genes coding for these proteins were cloned.

### (1) Extraction of mRNA

The rinderpest virus L strain that can be grown in Vero cells (rinderpest virus L 13 strain) was cultured, mRNA was extracted from the culture, and the cDNA was prepared from the mRNA according to the Okayama-Berg method (Okayama and Berg, Mol. Cell Boil., 2, 161-170, 1983; and Mol. Cell Boil., 3, 280-289, 1983).

More specifically, the rinderpest virus L 13 was used as an origin of the mRNA. The L 13 strain was obtained by fusing a rabbit lymphocyte infected with a rabbit-adapted passaged strain of the rinderpest virus (L-lapinized strain and F-Vero cells, under polyethylene glycol, and passaging 15 generations. Sub-confluent F-Vero cells in eight Petri dishes each having a diameter of 6 cm were inoculated with the L 13 strain at an m.o.i. of 1. The culture was passaged on the next day, and 24 hours after the passage, 20 µg/ml of actinonycin D was added to the culture to inhibit a synthesis of cellular mRNA and incubation was continued for an additional 5 hours. The recovered cells were centrifuged in 6 M of guanidium isothiocyanate (GTC), 5 mM of sodium citrate and 0.5% of Sarkosyl-5.7 M of CsCl and 0.1 M of EDTA at 35,000 rpm (150,000 XG) for more than 12 hours, according to the guanidinum-cesium chloride method, to recover the RNA (T. Maniatis et al., Molecular Cloning, p 196, 1982).

The thus-recovered RNA was applied to an oligo dT cellulose column, and the adsorbed RNA was eluted by 10 mM Tris-HCl (pH7.5) buffer containing 1 mM EDTA and 0.05% SDS to obtain a poly A(⁺) RNA according to a known procedure (T. Maniatis et al., Molecular Cloning, p 197, 1982).

### (2) Preparation of probes

According to the present invention, to confirm the presence of a target mRNA in the above-prepared poly A(⁺) RNA, and to screen the cDNA library as described hereinafter in detail, an H gene of a subacute sclerosing panencephalitis (SSPE) virus was used as a probe (designated as H gene probe or SSPE-H-cDNA probe) to detect and screen the gene coding for the H protein (H gene) of the rinderpest virus; and the F gene of the canine distemper virus (CDV) was used as a probe (designated as F gene probe or CDV-F-cDNA probe) to detect and screen the gene coding for an F protein (F gene) of the rinderpest virus. These genes were selected as probes because the above-mentioned SSPE virus and CDV, as well as the rinderpest virus, belong to the common genus Morbillivirus and these viruses are closely related to each other, and therefore, it is considered that the nucleotide sequences thereof will have a high homology.

The above-mentioned probes were prepared by obtaining an SSPE virus DNA and CDV DNA and labeling these DNA with a radioisotope ³⁵S (T. Maniatis et al., Molecular Cloning, p 109, 1982).

More specifically, a cDNA library was made from SSPE infected cells, and SSPE H cDNA was selected by the use of a measles virus H probe (Billeter et al. 1984, Virology 145 195). (DV H DNA was made according to Russel et al. (1985) (J. Gen. Virol, 66 433.). Each DNA thus-prepared was incubated with three unlabeled deoxyribonucleotides dATP, dGTP and dTTP, and a ³⁵S-labeled deoxyribonucleotide ³⁵S-dCTP-γS in the presence of E. coli DNA polymerase I in a Tris-HCL (pH7.8) buffer. The labeled DNA was then separated from unreacted ³⁵S-dCTP-γS by a Sephadex G25 column.

### (3) Detection of H protein mRNA and F protein mRNA in poly A(⁺) RNA

The poly A(⁺) RNA prepared as described above was denatured with glyoxal or formamide, and separated by Agarose gel electrophoresis, and the separated mRNAs were subjected to hybridization with the above-mentioned SSPE-H-cDNA probe or CDV-F-cDNA probe according to the conventional Northern hybridization method. As a result, a 1.96 kb mRNA which hybridizes with the SSPE-H-cDNA probe and a 2.2 kb mRNA which hybridizes with the CDV-F-cDNA were detected. This result suggests that the poly A(⁺) RNA preparation includes an mRNA coding for the H protein of the rinderpest virus and an mRNA coding for the F protein of the rinderpest virus.

### (4) Preparation of cDNA library

A cDNA library was prepared from the above-mentioned poly A(⁺) RNA preparation according to the Okayama-Berg method (Okayama H. and P. Berg, Mol. Cell Biol., 2, 161, 1982; and Mol. Cell Biol., 3, 280, 1983). As a vector, an oligo dT-tailed (T = 17) plasmid primer (pcDV-1) and an oligo dG-tailed (G = 12) pL-1 Hind III linker were used.

More specifically, 8 µg of the above-mentioned poly A(⁺) RNA, 2 µg of the dT-tailed plasmid primer (pCDV-1), 2 mM of four dNTPs including ³⁵S-dCTP, and 20.6 units of a reverse transcriptase (RTase) were incubated in 40 µl of a reaction mixture containing 100 mM of Tris-HCl (pH 8.3), 10 mM of MgCl₂ , 140 mM of Kcl, 2 mM of methylmercury hydroxide, 20 mM of mercaptoethanol, and 1 mM of RNase inhabitor at 42°C for 60 minutes, to form a cDNA-plasmid. To the reaction mixture, 3.5 mM of dCTP and 30 units of terminal transferase were added, resulting in the formation of 73.5 µl total volume of the reaction mixture. The reaction mixture was subjected to reaction at 37°C for 3 minutes, and 2.5 mM of EDTA containing 0.5% (final concentration) of SDS was added to the reaction mixture to terminate the reaction. The reaction mixture was extracted with chloroform and phenol, and the DNA was then precipitated with ethanol.

The thus-prepared cDNA-plasmid was digested with 20 units of HindIII in 40 µl of a buffer containing 10 mM of Tris-HCl (pH7.5), 50 mM of NaCl, 10 mM of MgCl₂ and 1 mM of dithiothreitol, at 37°C for 2 hours. Then, 120 mg of the digested cDNA-plasmid and 35 µg of the PL-1 linker were incubated in 40 µl of a reaction mixture containing 0.1 M NaCl, 10 mM Tris-HCl (pH7.8) and 1 mM EDTA, first at 65°C for 5 minutes and that of 42°C for 60 minutes, and the reaction mixture was then cooled to a room temperature to allow annealing. After the reaction mixture was cooled to 0°C, 0.3 µg of E. coli DNA ligase was then added to the reaction mixture, and the reaction mixture was incubated at 12°C overnight.

Then, 200 mg of E. coli DNA ligase, 0.450 units of RNase H and 0.8 units of DNA polymerase were added to the reaction mixture to make the total volume of the mixture 48 µl, and the temperature of the reaction mixture was then increased stepwise to 12°C, 15°, 20°C, and to 25°C, for 30 minutes at each temperature, resulting in digestion of an mRNA region, formation of a double-stranded cDNA and ligation of the resulting cDNA, to complete the construction of plasmids containing the cDNA.

The thus-obtained plasmids were used to transform Escherichia coli DH-5 and E. coli MC1061, and a cDNA library comprising 7,700 clones and a cDNA library comprising 7,300 clones (total 15,000 clones) were obtained from E. coli DN-5 and E. coli MC1061, respectively.

### (5) Screening of cDNA library

From among the above-mentioned 15,000 clones, 109 clones were screened by the colony hybridization method. As a result, a clone hybridizing with the SSPE-H-cDNA probe and a clone hybridizing with CDV-F-cDNA were obtained. These clones were designated as Escherichia coli DH-RVH and Escherichia coli DH-RVF respectively; and plasmids in these E. coli cells were designated as pDH-RVH and pDH-RVF. The size of cDNA insert (designated as RV-H) in the plasmid pDH-RVH was about 2.2 kb, and the size of the cDNA insert (designated as RV-F) in the plasmid pDH-RVF was about 1.6 kb. It was considered that these cDNA inserts contained approximately the entire coding regions for the H protein and F protein of the rinderpest virus, respectively.

The above-mentioned Escherichia coli DH-RVH was deposited with the Fermentation Research Institute Agency of Industrial Science and Technology (FRI), 1-1 Higashi 1-chome, Yatabe-shi, Ibaraki-ken, Japan, as FERM BP-1165 under the Budapest treaty on August 29, 1986. The above-mentioned Escherichia coli DH-RVF was deposited with the FRI as FERM BP-1164 under the Budapest treaty on August 29, 1986.

By repeating the same procedure as described above, a clone hybridizing with the SSPE-H-cDNA probe and a clone hybridizing with the SSPE-H-cDNA were obtained. These clones were designated as Escherichia coli DH-RVH-2 and Escherichia coli DH-RVF-2, respectively; and plasmids in these E. coli cells were designated as pDH-RVH-2 and pDH-RVF-2, respectively. The size of the cDNA insert (designated as RV-H-2) in the plasmid pDH-RVH-2 was about 3.2 kb, and the size of the cDNA insert (designated as RV-F-2) in the plasmid pDH-RVF-2 was about 2.4 kb. The RV-H-2 contained an entire coding region for the H protein of the rinderpest virus, and the RV-F-2 is considered to contain an entire coding region for the F protein of the rinderpest virus.

The above-mentioned Escherichia coli DH-RVH-2 was deposited with the FRI as FERM BP-1319 under the Budapest treaty on March 24, 1987. The above-mentioned Escherichia coli DH-RVF-2 was deposited with the FRI as FERM BP-1318 under the Budapest treaty on March 24, 1987.

### (6) Restriction enzyme cleavage map

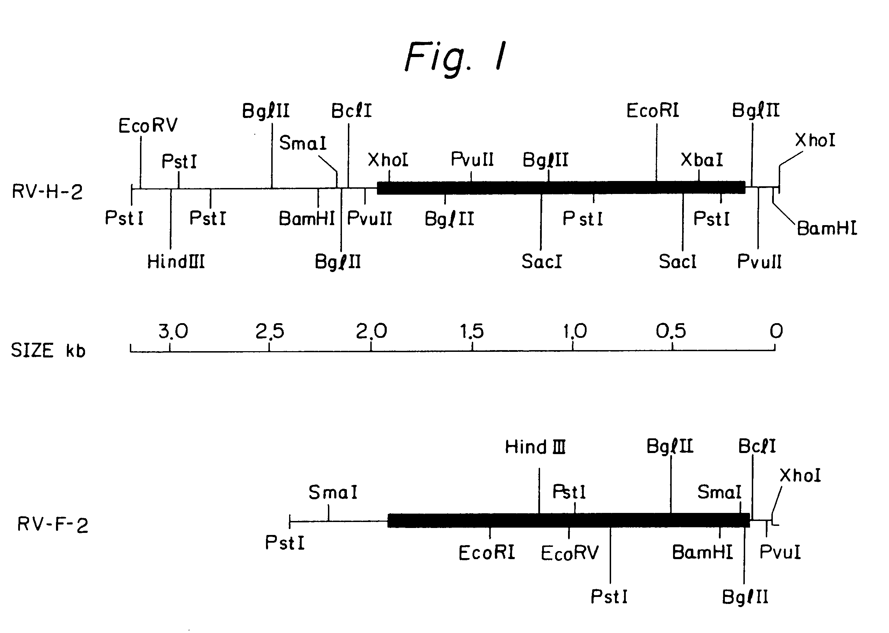

The restriction enzyme cleavage sites of the above-mentioned cDNA inserts were analyzed to determine restriction enzyme cleavage maps, according to a conventional procedure. Figure 1 represents the restriction enzyme cleavage maps for the cDNA inserts RV-H-2 and RV-F-2.

### (7) Determination of nucleotide sequences of cDNAs coding for H protein and F protein

The plasmids pDH-RVH and pDH-RVH-2 were digested with restriction enzymes BamHI, PvuII, PstI, EcoRI, HindIII, and SmaI, and DNA fragments of several hundred base pairs in length were separated. Each DNA fragment was inserted to a polylinker site of a phage M13 DNA, and the nucleotide sequence was determined according to the Sanger dideoxy chain termination method. The result is shown in Figs. 2-1 to 2-4.

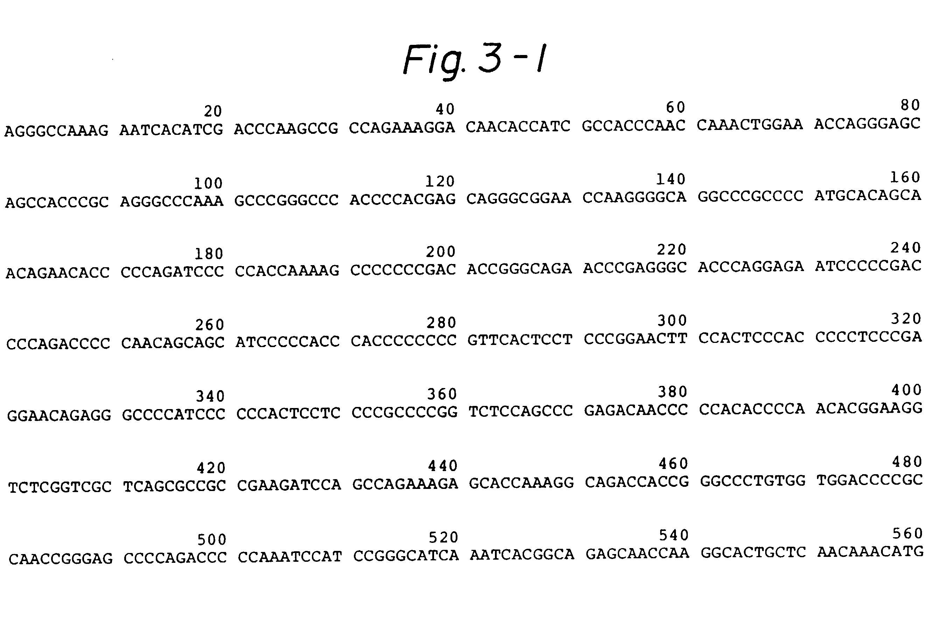

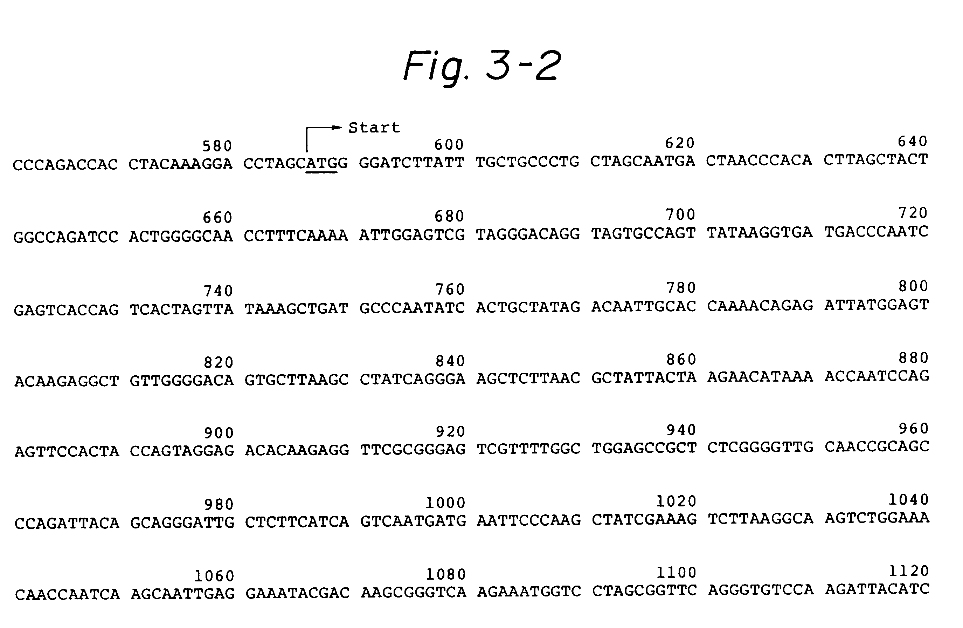

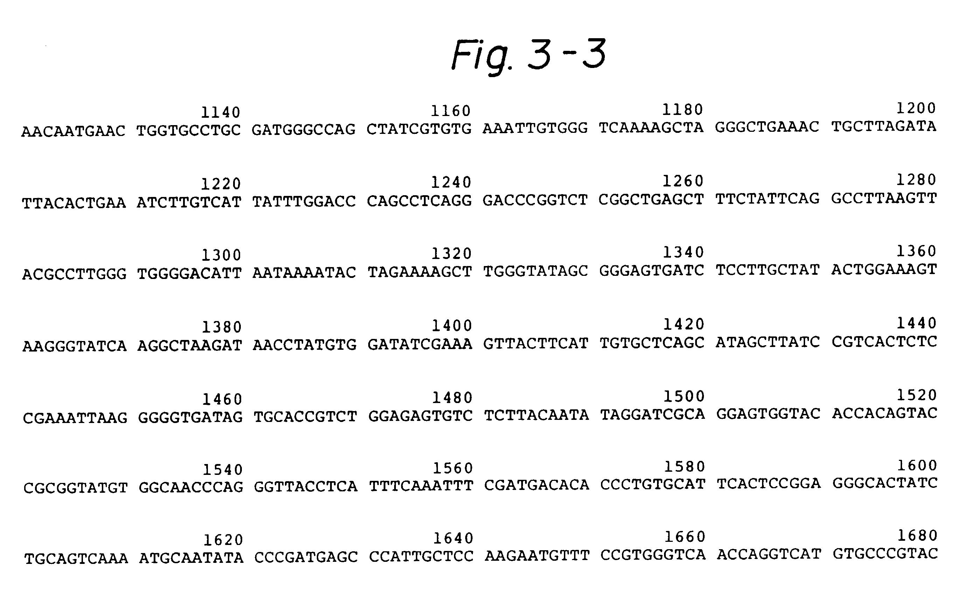

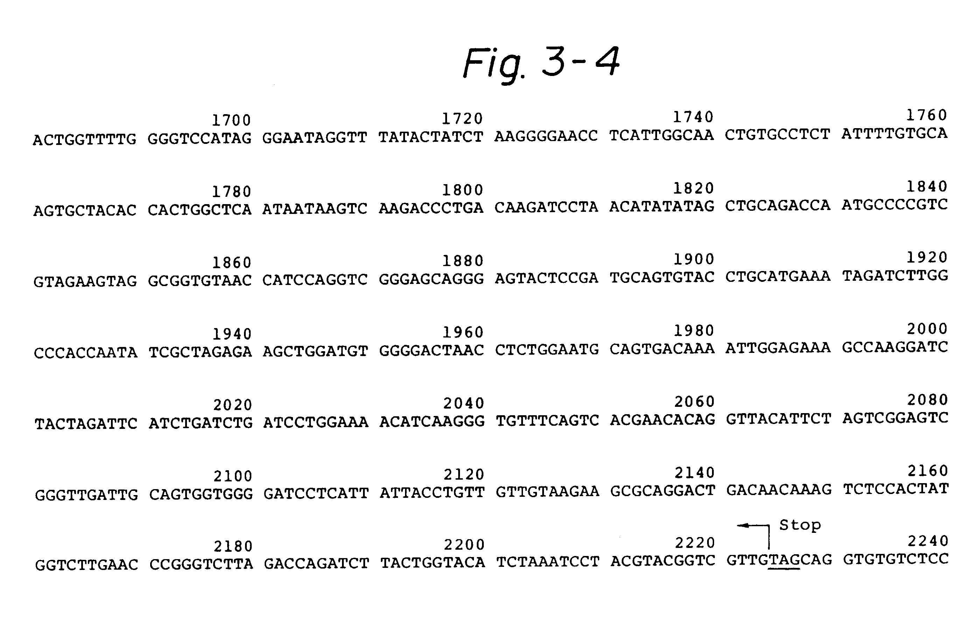

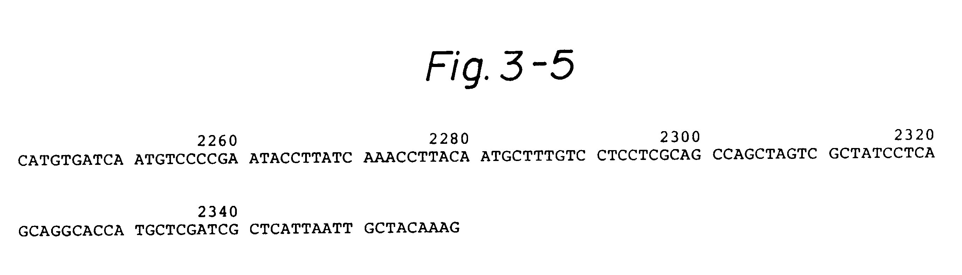

According to a similar procedure, nucleotide sequence coding for F protein was determined. The result is shown in Figs. 3-1 to 3-5.

### (8) Expression of the gene

COS7 cells were cultured in a cell culture chamber in 1 ml of Eagl's minimum essential medium containing 10% felal calf serum at 37°C for 24 hours. The DNA of the plasmid pDH-RVH-2 was coprecipitated with calcium phosphate, and 1 µg of the precipitated DNA was transfected to the cultured COS7 cells. The cells were then treated with a DMEM medium containing 10% dimethylsulfoxide and 5% fetal calf serum. Fresh medium was added to the cells, and culturing was carried out at 37°C for two to three days. The cells were then fixed with acetone, and were observed by an indirect fluorescent antibody technique using an antibody specific to the measles virus. In this technique, an antigen H reactive with the antibody was observed on the cells. In same manner, it was confirmed that the pDH-RVF and pDH-RVF-2 expressed an antigen F.

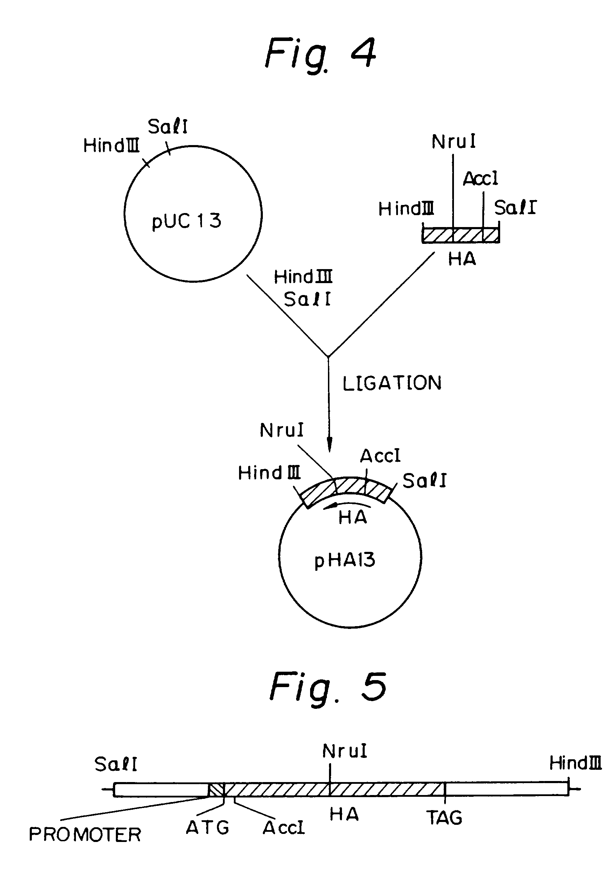

### Example 2 Construction of plasmid pHA13 (cloning of vaccinia virus hemagglutinin gene (HA)) (Figs. 4 and 5)

Virion of the vaccinia virus WR was purified by a conventional procedure, and the virion was suspended in 50 mM Tris-HCl (pH7.4) containing 1 mM EDTA and 0.5% sodium lauryl sulfate, 250 to 1000 µg/ml proteinase K was then added to the suspension, and incubation was carried out at 37°C overnight. The suspension was extracted three times with phenol/chloroform (1:1) saturated with 10 mM Tris-HCl (pH8.0) containing 1 mM EDTA, and a virus DNA was precipitated by ethanol. The DNA was dissolved in 10 mM Tris-HCl (pH8.0) containing 1 mM EDTA, digested with HindIII, and the digestion product was subjected to agarose gel electrophoresis to isolate a HindIII fragment of about 50 kpb. The HindIII fragment was digested with SalI in a high salt concentration buffer, and a HindIII-SalI fragment of about 1.5 kbp present in 3′terminal region in the HindIII A fragment was isolated by agarose gel electrophoresis.

On the other hand, a plasmid pCU13 was digested with HindIII in a middle salt concentration buffer, and then with SalI in a high salt concentration buffer to form a linearized plasmid, which was then isolated by agarose gel electrophoresis.

The above-mentioned HindIII-SalI fragment and the linearized plasmid were ligated using T4 DNA ligase in a ligation buffer, and the ligation mixture was used to transform E. coli JM109. A plasmid was extracted from each clone by a rapid alkaline extraction method, and a desired plasmid was selected by restriction enzyme analysis, and designated as plasmid pHA13.

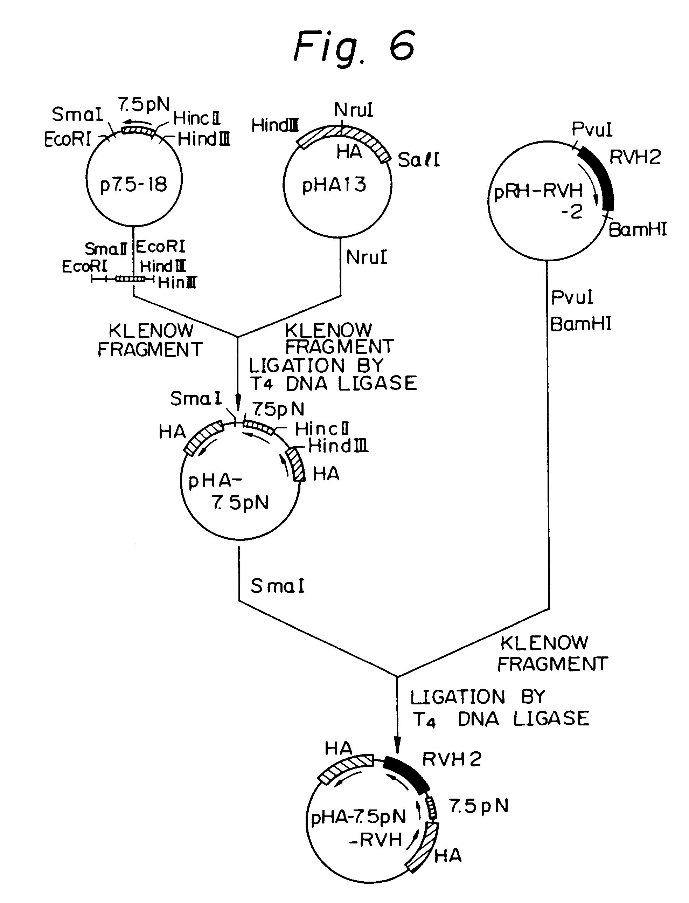

### Example 3 Construction of recombinant plasmid pHA-7.5pN-RVH containing 7.5K promoter (Fig. 6)

A plasmid used for integration of the rinderpest virus hemagglutinin gene (RVH) linked with a 7.5K promoter into a vaccinia virus gene was constructed as follows.

To clone a promoter of a vaccinia virus 7.5K protein gene (7.5K promoter or 7.5pN), virus DNA was extracted from virion of a vaccinia virus WR. According to a process described by Venkatesan et al., J. Virol., 33, 738-745, 1981, the extracted DNA was digested with SalI to isolate 0.9 kbp DNA fragment, which was then cloned to SalI site of plasmid pUC18. Next, the plasmid thus prepared was digested with RsaI and HincII to obtain a 0.26 kbp RsaI-HincII fragment, which was then inserted to a plasmid pUC18 to construct a plasmid containing a 7.5K promoter, which was designated as a plasmid p7.5-18.

The plasmid p7.5-18 was digested with EcoRI and HindIII in a medium salt concentration buffer to excise a DNA fragment of about 0.29 kbp containing a 7.5K promoter (7.5pN), and the fragment was isolated by agarose gel electrophoresis. On the other hand, the plasmid pHA13 constructed in Example 2 was cleaved with NruI to form a linearized plasmid. Each were then treated with a Klenow fragment in the presence of dTTP, dCTP, dATP and dGTP in the nick-translation buffer to blunt the ends thereof. These DNA fragments were ligated using T4 DNA ligase, and the ligation mixture was used to transform E. coli JM109 to obtain plasmids. The plasmids were subjected to restriction enzyme analysis to select a plasmid which contains the vaccinia virus hemagglutinin gene (HA) and a 7.5K promoter (7.5pN) in the same orientation, and was designated as plasmid pHA-7.5pN.

Next, a plasmid pDH-RVH-2 containing RVH-2 was digested with PvuI and BamHI to obtain a DNA fragment of about 2 kpb containing RVH-2, and then was treated with a Klenow fragment to blunt the ends thereof, as described above. On the other hand, the above-mentioned plasmid pHA-7.5pN was digested with SmaI in a SmaI buffer to linearize the plasmid. The above-mentioned DNA fragment from pDH-RVH-2 and the linearized plasmid pHA-7.5pN were ligated using a T4 DNA ligase, the ligation mixture was used to transform E. coli JM109, and plasmids were extracted from the transformants. The plasmids were subjected to restriction enzyme analysis to select a plasmid wherein RVH-2 and 7.5 pN have been inserted into the HA in the same orientation, and was designated as plasmid pHa-7.5pN-RVH.

### Example 4 Cloning of ATI promoter

5 x 10⁸ of Vero cells (cells derived from renal cells of African green monkey) were infected with cowpox virus CPRO6 at an m.o.i. of 0.2, and the virus was recovered two days after the infection when the cytotoxicity became remarkable. The virus was then purified by sucrose density-gradient centrifugation to obtain about 3 mg of the virus.

Three mg of the purified virus was digested with 1 mg/ml of proteinase K at 37°C overnight in the presence of 0.5% sodium dodecyl sulfate (SDS) and 1 mM of ethylenediaminetetraacetic acid sodium salt (EDTA). The digest was extracted three times with phenol/chloroform and three times with ethyl ether to eliminate proteins. To the extracted digest was added 1/10 volume of 3 M sodium acetate and two volumes of isopropanol, and the mixture was stirred with a glass rod to recover flocculated DNA on the rod. The recovered DNA was dissolved in a TE buffer (10 mM Tris-HCl, pH 8.0, 1 mM EDTA) and 180 µg of DNA was obtained.

Ten µg of the cowpox virus DNA prepared as above was cleaved with a restriction endonuclease HindIII or SalI in a buffer containing 10 mM Tris-HCl (pH7.5), 60 mM NaCl and 7 mM MgCl₂ , or a buffer containing 10 mM Tris-HCl (pH 7.5), 150 mM NaCl, and 7 mM MgCl₂ , respectively, at 37°C for 120 minutes. The HindIII-cleaved DNA was mixed with a plasmid pUC18 which had been cleaved with HindIII; and the SalI-cleaved DNA was mixed with a plasmid pUC13 which had been cleaved with SalI, and each of the mixtures was subjected to ligation in a buffer containing 66 mM Tris-HCl (pH 7.5), 5 mM MgCl₂ , 5 mM dithiothreitol and 1 mM ATP using a T4 DNA ligase at 16°C for 16 hours. The ligation mixture was used to transform E. coli JM103 or E. coli JM109 to prepared genomic DNA libraries of the cowpox virus. Each recombinant plasmid contained a genomic DNA fragment of about 1 Kb to 25 Kb.

The plasmids containing a cowpox virus DNA fragment were transfected to CV-1 cells which had been infected with vaccinia virus, and expression of the A-type inclusion body gene in the transfected cells was confirmed by Western blotting to identify DNA containing an A-type inclusion body gene.

Namely, 3 x 10⁵ of CV-1 cells (cells from monkey kidney) were infected with a vaccinia virus WR strain at an m.o.i. of 50 and allowed to stand for one hour. A plasmid containing the cowpox virus DNA was extracted from 2 ml of E. coli culture by an alkaline extraction method, and 10 µg of the plasmid DNA was transfected to cells previously infected with the vaccinia virus. After standing for 30 minutes at room temperature, 3 ml of a medium containing 5% fetal calf serum was added to the culture, which was then incubated at 37°C for 5 hours, and after an exchange of the medium incubated at 37°C overnight. After sonication of the cell suspension, the sonicate was subjected to SDS-polyacrylamide gel electrophoresis, and the gel was subjected to Western blotting. That is, proteins on the gel were electrophoretically transferred to a nitrocellulose filter. The nitrocellulose filter was treated with a buffer containing 10 mM Tris-HCl (pH7.5), 0.15 M NaCl and 5% bovine serum albumin, and reacted with an anti-A-type inclusion body antibody in a buffer containing 10 mM Tris-HCl (pH7.5) and 0.15 M NaCl at 37°C for one hour. Next, the filter was reacted with an anti-rabbit IgG antiserum conjugated with peroxidase at 37°C in the same buffer as described above, and finally, treated with a 10 mM Tris-HCl (pH7.5) buffer containing 0.01% hydrogen peroxide, 0.5 mg/ml 4-chloronaphthol and 0.15 M NaCl to develop an A-type inclusion body gene expression product. As a result, SalI fragment of 22 kb was found to contain the A-type inclusion body gene. This fragment was designated as 0804, and the plasmid containing this fragment was designated as p0804.

The plasmid p0804 was digested with SalI, and the resulting SalI fragment was cleaved with a restriction endonuclease KpnI, SphI, PstI or SacI. Each fragment thus obtained was ligated with a plasmid pUC 18 or plasmid pUC 19, which had been cleaved with a corresponding restriction endonuclease to obtain recombinant plasmids pB6, pB20, pB23, pC3, pC6, etc.

These plasmids were then tested for the presence of an A-type inclusion body gene therein, and it was confirmed that a KpnI-SphI fragment of 9.1 kb in the plasmid pB23, and a SacI-SalI fragment of 8.9 kb in the plasmid pC3 included an entire gene including its promoter for a major protein of A-type inclusion body; and a SacI-SalI fragment of 6.2 kb in the plasmid pC6 included a part of a gene including a promoter thereof for a major protein of A-type inclusion body.

Accordingly, in the present invention any of the above-mentioned plasmids can be used as an ATI promoter (ATIp) source.

Note, Escherichia coli JM109-B23, containing the plasmid pB23, was deposited with the Fermentation Research Institute Agency of Industrial Science and Technology (FRI), 1-1 Higashi 1-chome Tsukuba-shi, Ibaraki-ken, Japan as FERM P-8971, on September 19, 1986, and transferred to the international deposition under the Budapest Treaty on the International Recognition of the Deposit of Microorganisms for the Purpose of Patent Procedure as FERM BP-1459 on September 1, 1987.

The nucleotide sequence of the ATI promoter is disclosed in E.P. Publication No. 0261925.

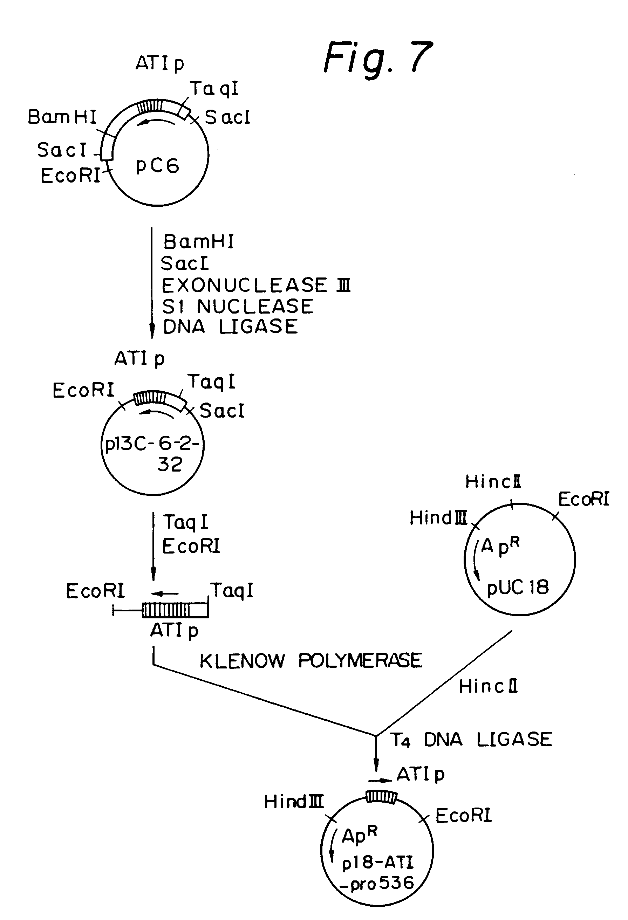

### Example 5 Construction of plasmid p13C-6-2-32 (Fig. 7)

The plasmid pC6 constructed in Example 4 was digested with SacI in a low salt concentration buffer, and then with BamHI in a high salt concentration buffer. After phenol extraction and ethanol precipitation, the DNA was shortened with exonuclease III by about 3.3 kbp in the direction from the BamHI end to the ATI promoter region in an exonuclease III buffer. The shortened DNA was treated with Sl nuclease in an Sl nuclease buffer, to eliminate a single strand at the ends of the DNA and blunt the ends thereof. Next, the DNA was re-ligated with a T4 DNA ligase to circularize the DNA, and the ligation mixture was used to transform E. coli JM109. Plasmids were extracted from the resulting transformants, and the plasmids were analyzed with restriction enzymes to select a desired plasmid, designated as plasmid p13C-6-2-32.

### Example 6 Construction of plasmid p18-ATI-pro536 (Fig. 7)

A cloning site for insertion of an exogenous gene was introduced downstream of the ATI promoter as follows.

A plasmid p13C-6-2-32 was digested with TaqI and EcoRI in a high salt concentration buffer, and a DNA fragment containing an ATI promoter was treated with a Klenow polymerase to blunt the ends thereof. On the other hand, a plasmid pUC18 was digested with HincII in a high salt concentration buffer to linearize the plasmid. The DNA fragment containing the ATI promoter and the linearized plasmid were ligated using a T4 DNA ligase, and the ligation mixture was used to transform E. coli JM109. Plasmids resulting from the transformants were analyzed by restriction enzymes to select a plasmid wherein the ATI promoter was oriented from the HindIII site to the EcoRI site, and this plasmid was designated plasmid p18-ATI-pro536.

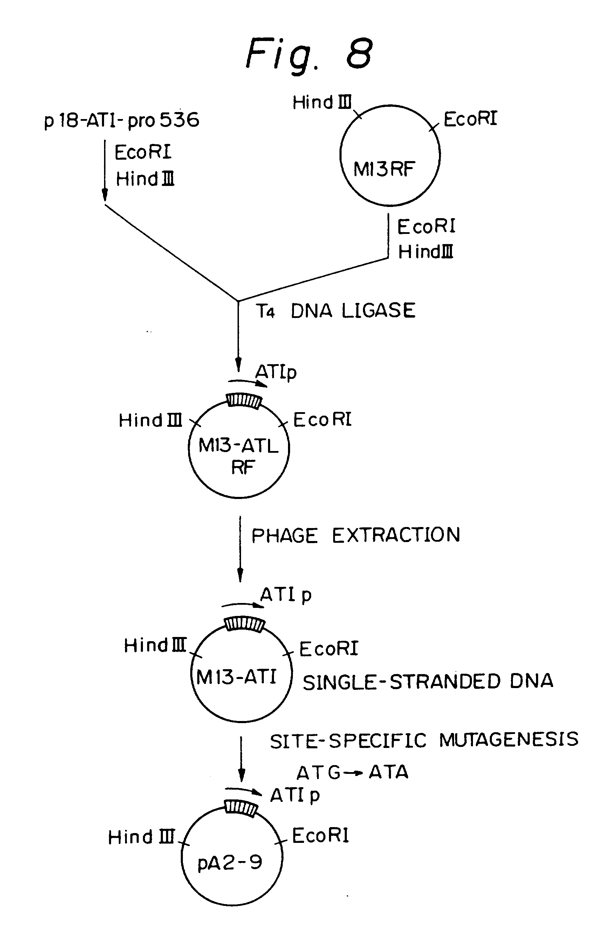

### Example 7 Construction of pA2-9 (Fig. 8)

The plasmid p18-ATI-pro536 contains an ATG codon immediately downstream of the ATI promoter region, and since this ATG is a start codon for the ATI protein structure gene, the presence of this ATG is undesirable when the ATI promoter is used to express an exogenous gene. Accordingly, the ATG was converted to ATA, as follows.

The plasmid p18-ATI-pro536 was digested with HindIII in a medium salt concentration buffer, followed by EcoRI digestion in a high salt concentration buffer, to obtain a DNA fragment containing the ATI promoter. On the other hand, a phage M13RF (reprication type) DNA was digested with HindIII and EcoRI as described above. The DNA fragment containing the ATI promoter and the phage DNA fragment were ligated using a T4 DNA ligase, and the ligation mixture was used to transfect E. coli JM109.
A phage DNA was extracted from the transfected E. coli, by an alkaline method, and subjected to restriction enzyme analysis to select a phage containing the ATI promoter. The selected phage was designated M13-ATI.

E. coli JM109 was transfected with the M13-ATI and cultured to obtain a supernatant, and from the supernatant, a single-stranded phage DNA was recovered. The recovered phage DNA was purified by ethanol precipitation. Next, site-directed mutagenesis was carried out with a primer nucleotide 5′-AATAAATAGAGGTCACGAACC-3′ using a kit "Origonucleotide Site-Directed Mutagenesis in M13" (Anglian Bio-technology Inc.) The change of the nucleotide was confirmed by sequencing using dideoxy chain termination method, to obtain a desired mutant plasmid designated pA2-9.

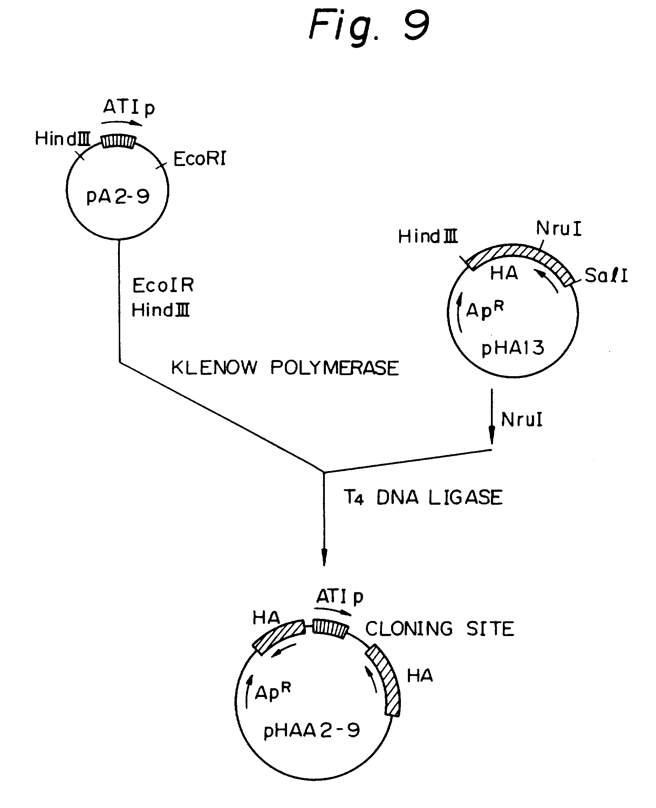

### Example 8 Construction of pHAA2-9 (Fig. 9)

The plasmid pA2-9 was digested with HindIII in a medium salt concentration buffer, and then with EcoRI in a high salt concentration buffer, and the EcoRI-HindIII fragment containing ATI promoter was treated with a Klenow polymerase to obtain a blunt-ended EcoRI-HindIII fragment. On the other hand, the plasmid pHA13 constructed in Example 2 was digested with NruI to obtain a linearized plasmid DNA. The DNA fragment containing the ATI promoter and the linearized plasmid DNA were ligated using a T4 DNA ligase, and the ligation mixture was used to transform E. coli. Plasmids were extracted from the transformants, and the plasmids were subjected to restriction enzyme analysis to select a plasmid wherein the ATI promoter has been inserted into a vaccinia virus hemaglutinin gene (HA) in an opposite orientation. The selected plasmid was designated pHAA2-9.

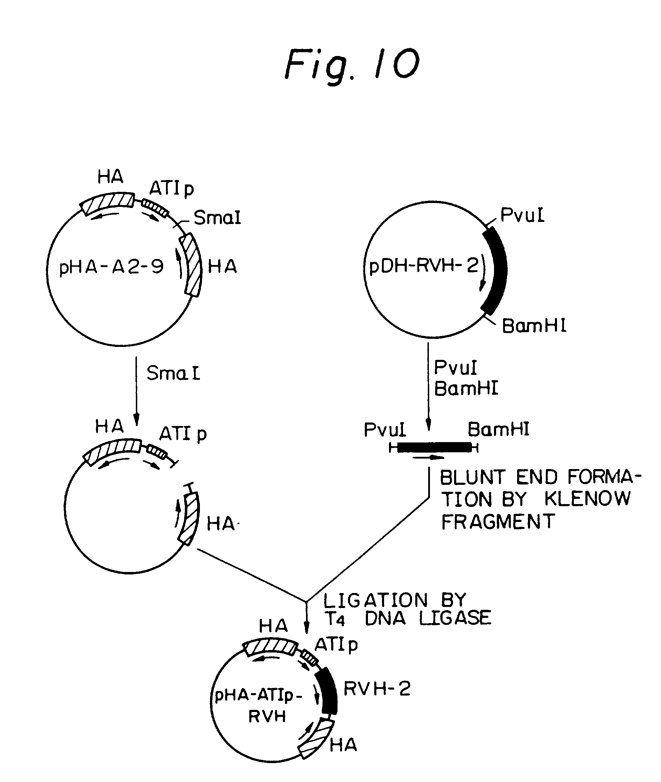

### Example 9 Construction of plasmid pHA-ATIp-RVH (Fig. 10)

The plasmid pDH-RVH-2 constructed in Example 1 was digested with PvuI and BamHI, and a DNA fragment containing the rinderpest virus hemagglutinin gene (RVH-2) was isolated by agarose gel electrophoresis, and then treated with a Klenow fragment to blunt the ends thereof. On the other hand, the plasmid pHAA2-9 constructed in Example 8 was cleaved with SmaI in a SmaI buffer to obtain a linearized plasmid DNA. The DNA fragment containing RVH-2 and the linearized plasmid DNA were ligated with a T4 DNA ligase, and the ligation mixture was used to transform E. coli DH5. Plasmids were recovered from resulting transformants, and the plasmids were subjected to restriction enzyme analysis to select a plasmid wherein RVH-2 and the ATI promoter have been inserted into the vaccinia virus hemagglutinin gene (HA) wherein the ATP promoter and RVH-2 have the same orientation. This plasmid was designated pHA-ATIp-RVH.

### Example 10 Synthesis of DNA corresponding to a portion upstream of XhoI site of RVH

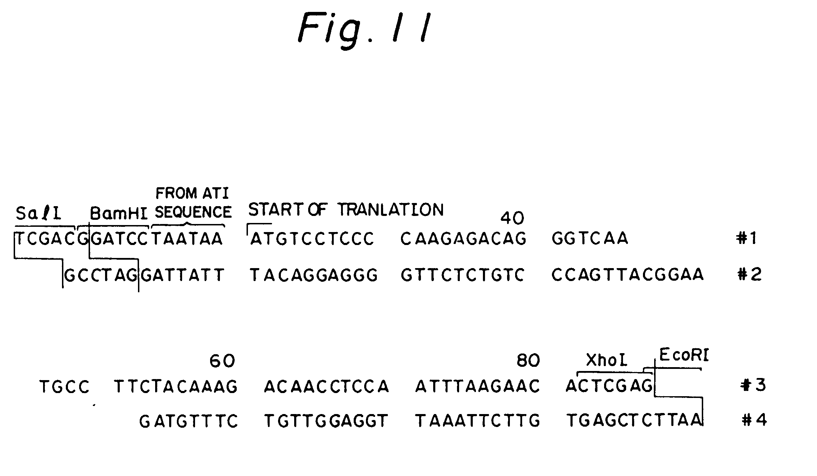

Nucleotide sequences near the start codon ATG in vaccinia virus latter protein genes are highly preserved, and have the sequence TAAATG..... It is considered that this sequence is important for transcription of a latter protein gene to mRNA. Accordingly, a DNA corresponding to an upstream portion of the rinderpest virus hemagglutinin gene was designed to include the above-mentioned sequence upstream of the hemagglutinin gene. Moreover, for a convenient insertion of the DNA, restriction enzyme cleavage sites were provided at both ends of the DNA. Since the DNA to be synthesized is long, four oligonucleotides, which form the desired DNA, were used. The nucleotide sequences of these oligonucleotides are set forth in Fig. 11.

These origonucleotides were synthesized by a phosphoamidite method using a DNA synthesizer (Applied Biosystems). The synthesized oligonucleotides were purified, and after annealing and ligation, the DNA was cloned in a plasmid pUC9 to form the plasmid pA-RVH. The correct nucleotide sequence of the synthetic DNA was confirmed by cloning the DNA fragment into the M13mp18 phage and determining the nucleotide sequence using an M13 Sequence kit (Takara).

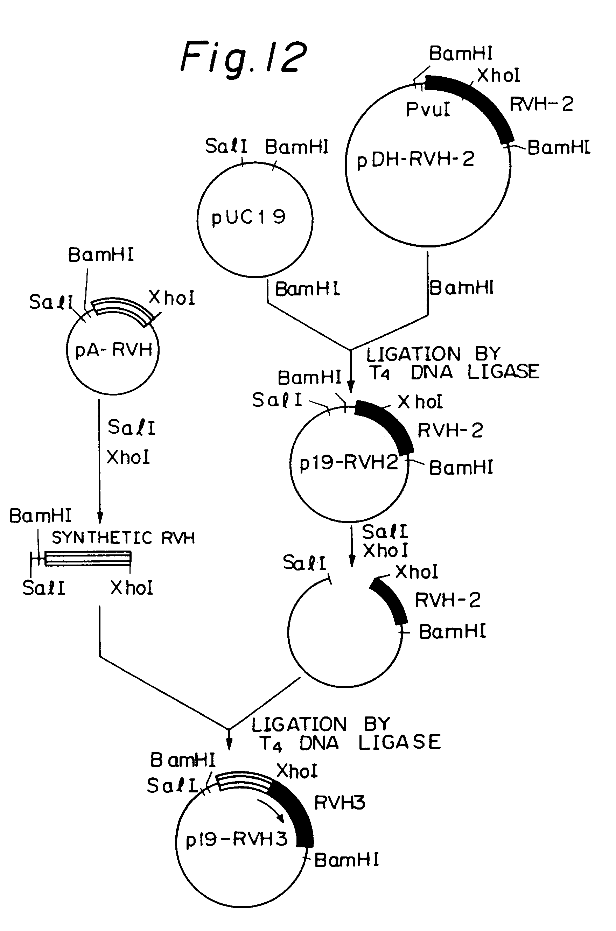

### Example 11 Construction of p19-RVH3 by joining synthetic DNA and RVH-2 (Fig. 12)

The plasmid pDH-RVH-2 constructed in Example 1 was digested with BamHI in a high salt concentration buffer to obtain a SalI-XhoI DNA fragment of about 2.2 kbp containing the rinderpest virus hemagglutinin gene (RVH-2). On the other hand, a commercially available plasmid pUC19 was digested with BamHI, as described above, to form a linearized vector DNA. The DNA fragment containing RVH-2 and the vector DNA were ligated using a T4 DNA ligase, to construct a desired plasmid p19-RVH-2. The plasmid p19-RVH-2 was digested with SalI and XhoI in a high salt concentration buffer to delete a portion of the RVH-2 upstream of the XhoI site. The resulting linealized plasmid DNA was ligated with the above-mentioned SalI-XhoI DNA fragment containing a synthetic DNA fragment using a T4 DNA ligase, and the ligation product was used to transform E. coli TB-1.

Plasmids were isolated from the transformants and subjected to restriction enzyme analysis, to select a plasmid containing the synthetic DNA fragment joined with RVH-2 at the XhoI site in a correct orientation. Further, the correct junction was confirmed by digesting the resulting plasmid with SalI and SacI to obtain a SalI-SacI fragment, cloning the fragment in a M13mp18 phage, and determining the nucleotide sequence. The reconstructed gene coding for the rinderpest virus hemagglutinin was designated RVH-3, and the obtained plasmid was designated p19-RVH-3.

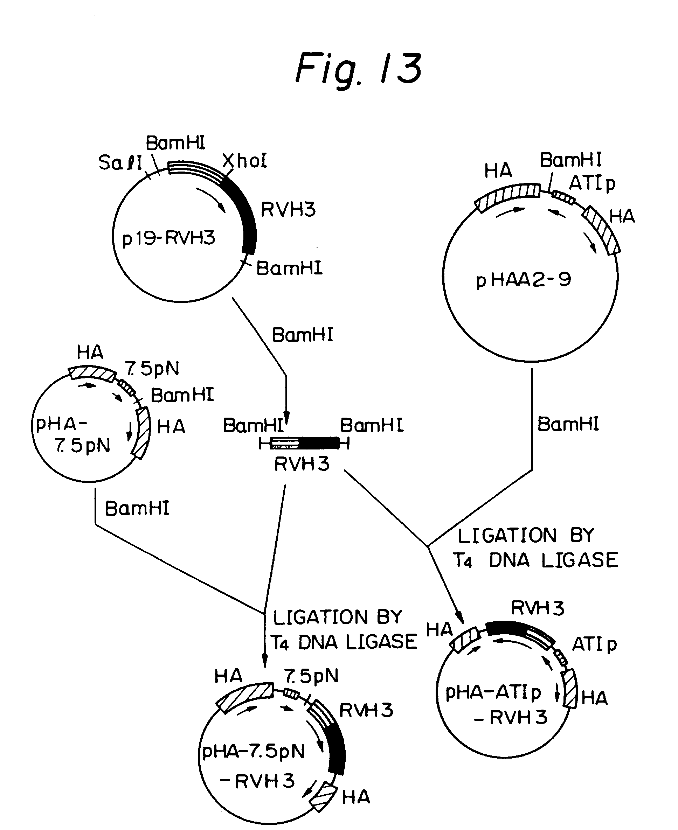

### Example 12 Construction of plasmid pHA-ATIp-RVH-3 (Fig. 13)

A plasmid p19-RVH-3 was digested with BamHI in a high salt concentration buffer to obtain a DNA fragment containing the above-mentioned modified rinderpest virus hemagglutinin gene (RVH-3). On the other hand, the plasmid pHAA2-9 constructed in Example 8, which contains an ATI promoter inserted in a vaccinia virus hemagglutinin gene, was digested with BamHI in a high salt concentration buffer to obtain a linearized plasmid. The DNA containing RVH-3 and the linearized plasmid were ligated using a T4 DNA ligase in a ligation buffer, and the ligation mixture was used to transform E. coli TB-1. Plasmids were isolated from the transformants and subjected to restriction enzyme analysis, to select a plasmid wherein the modified gene RVH3 has been inserted downstream of and in the same orientation as the ATI promoter. The selected plasmid was designated pHA-ATIp-RVH-3.

### Example 13 Construction of plasmid pHA-7.5pN-RVH-3 (Fig. 13)

The plasmid p19-RVH-3 was digested with BamHI in a high salt concentration buffer to obtain a DNA fragment containing the above-mentioned modified rinderpest virus hemagglutinin gene (RVH-3). On the other hand, the plasmid pHA-7.5pN constructed in Example 3, which contains a 7.5K promoter (7.5pN) inserted in a vaccinia virus hemagglutinin gene, was digested with BamHI in a high salt concentration buffer to obtain a linearized plasmid. The DNA containing RVH-3 and the linearized plasmid were ligated using a T4 DNA ligase in a ligation buffer, and the ligation mixture was used to transform E. coli TB-1. Plasmids were isolated from the transformant and subjected to restriction enzyme analysis, to select a plasmid wherein the modified gene RVH-3 has been inserted downstream of and in the same orientation as the 7.5pN. The selected plasmid was designated pHA-7.5pN-RVH-3.

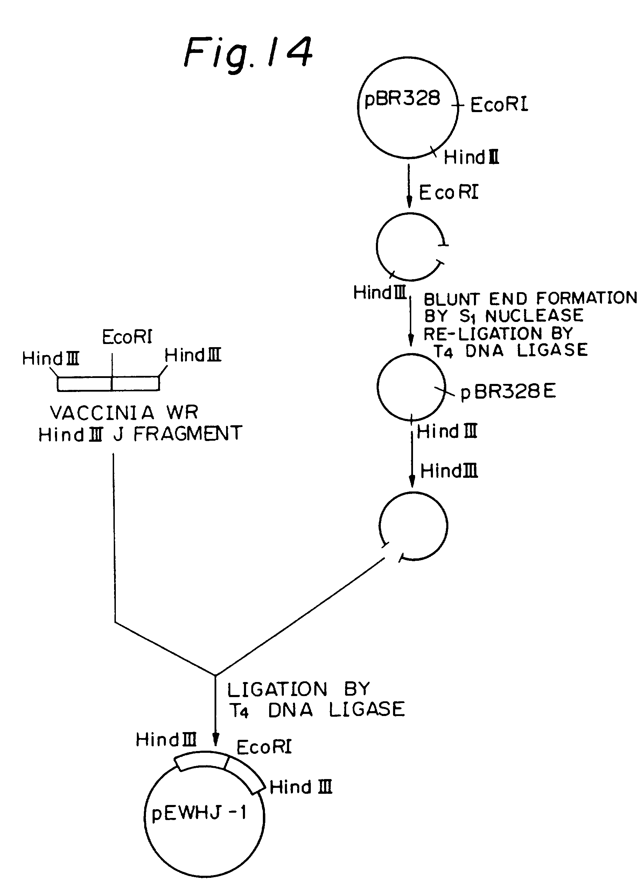

### Example 14 Cloning of vaccinia virus thymidine kinase gene (TK) (Fig. 14)

A vaccinia virus WR was cultured in RK13 cells, and virion was purified by a sucrose density gradient method. Viral DNA was extracted and digested with HindIII, and the digestion product was subjected to 0.6% agarose gel electrophoresis to isolate a J fragment. It was found that the J fragment contained a thymidine kinase gene, and that the thymidine kinase gene contained, at approximately a central region thereof, an EcoRI site which is unique in a J fragment.

On the other hand, to delate the EcoRI site in a commercially available plasmid pBR328, the plasmid pBR328 was digested with EcoRI in a high salt concentration buffer, and the linearized plasmid DNA was recovered and treated with S1 nuclease in an S1 nuclease buffer to blunt the ends thereof. Next, the DNA was recovered and re-ligated using a T4 DNA ligase to obtain a modified plasmid pBR328-E wherein the EcoRI site has been eliminated.

The plasmid pBR328-E was digested with HindIII in a medium salt concentration buffer to obtain a linearized plasmid. This linearized plasmid was ligated with the above-mentioned HindIII J fragment using a T4 DNA ligase, and the ligation mixture was used to transform E. coli HB101. Plasmids were recovered from the transformants and subjected to restriction enzyme analysis, to select a plasmid wherein the HindIII J fragment has an orientation opposite to the orientation of the Amp^{r} gene of pBR328. This plasmid was designated pEWHJ-1.

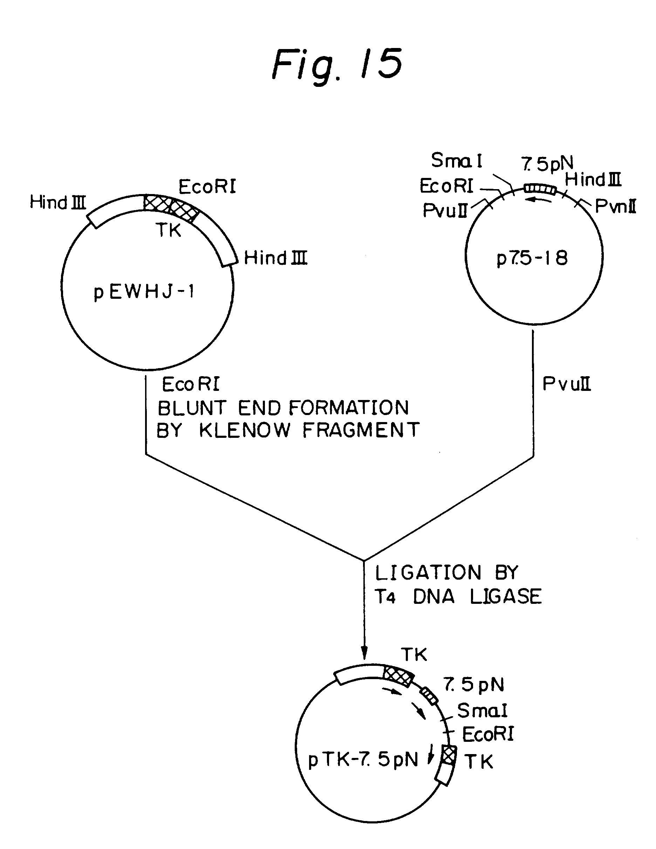

### Example 15 Construction of plasmid pTK-7.5pN (Fig. 15)

The plasmid pTK-7.5pN was constructed by insertion of a 7.5K promoter into the thymidine kinase gene in pEWHJ-1, as follows.

Since the plasmid pEWHJ-1 has a unique EcoRI site in the thymidine kinase gene at a central region thereof, the plasmid pEWHJ-1 was digested with EcoRI in a high salt concentration buffer, and a linearized plasmid DNA was recovered and treated with a Klenow fragment to blunt the ends thereof. On the other hand, a plasmid p7.5-18 was digested with PvuII, and a DNA fragment containing a 7.5K promoter and polylinker was isolated by agarose gel electrophoresis. This DNA fragment and the above-mentioned linearized plasmid DNA were ligated using a T4 DNA ligase, and the ligation mixture was used to transform E. coli TB-1. Plasmids were recovered from the transformants and subjected to restriction enzyme analysis, to select a plasmid wherein a 7.5K promoter and a thymidine kinase gene have the same orientation.

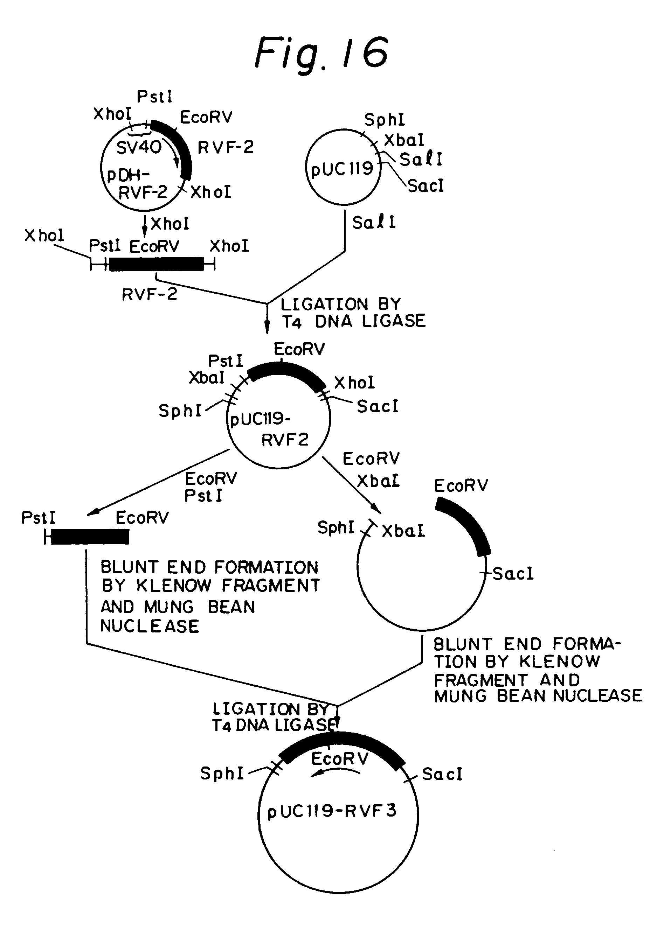

### Example 16 Construction of plasmid pUC119-RVF-3 (Fig. 16)

For an efficient expression of an exogenous gene, preferably the distance between the promoter and the exogenous gene is as short as possible, and there is no excess sequence near the ATG start codon. Accordingly, a SV40 sequence upstream of the rinderpest virus fusion protein gene (RVF-2) was removed, as follows.

First, a plasmid pDH-RVF-2 was digested with XhoI in a high salt concentration buffer to obtain a DNA fragment containing RVF-2. On the other hand, a plasmid pUC119 was digested with SalI in a high salt concentration buffer to obtain a linearized plasmid DNA. The DNA fragment containing RVF-2 and the linearized plasmid DNA were ligated using a T4 DNA ligase, and the ligation mixture was used to transform E. coli DH5. Plasmids were recovered from the transformants and subjected to restriction enzyme analysis, to select a plasmid containing RVF-2. This plasmid was designated pUC119-RVF-2.

Next, the plasmid pUC119-RVF-2 was digested with PstI and EcoRV to obtain a Pst-EcoRV DNA fragment of about 1.3 kb containing the upstream half of RVF-2. On the other hand, the plasmid pUC119-RVF-2 was digested with EcoRV in a medium salt concentration buffer, and then with XbaI in a high salt concentration buffer, to obtain a linearized plasmid DNA containing the downstream half of RVF-2. Both DNA fragment were treated with Mung bean nuclease to blunt the ends thereof, and ligated with a T4 DNA ligase. The ligation mixture was used to transform E. coli DH-5. Plasmids were isolated from the transformants and subjected to restriction enzyme analysis, to select a plasmid consisting of the above-mentioned two fragments joined to each other at the EcoRV site and having the same orientation. The reconstructed DNA coding rinderpest virus fusion protein gene was designated RVF-3. This plasmid was designated pUC119-RVF-3.

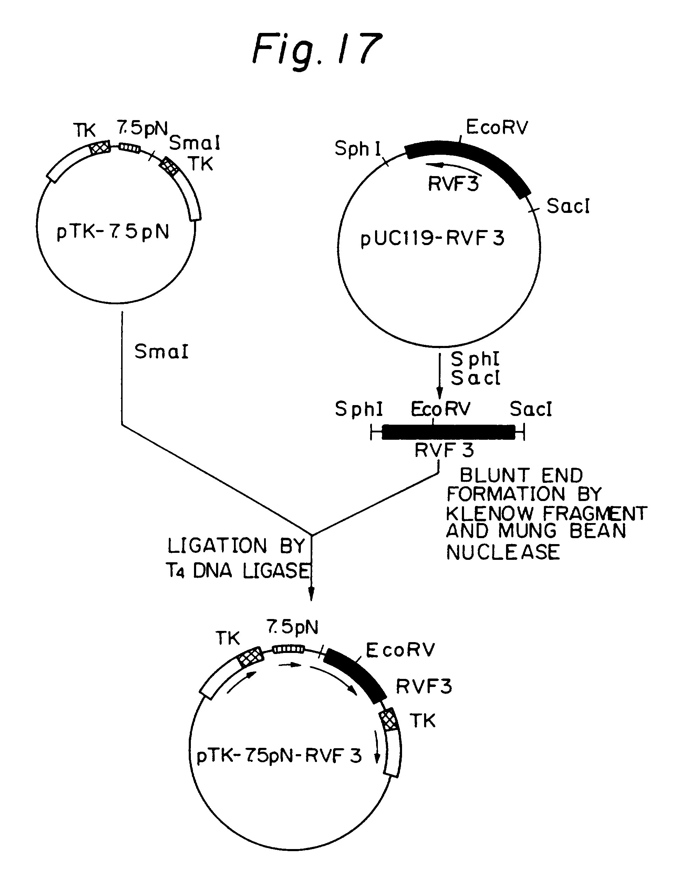

### Example 17 Construction of plasmid pTK-7.5pN-RVF-3 (Fig. 17)

The plasmid pUC119-RVF-3 was digested with SacI in a low salt concentration buffer, and the with SphI in a high salt concentration buffer, to obtain a DNA fragment containing RVF-3. This fragment was treated with Mung bean nuclease and a Klenow fragment to blunt the ends thereof. On the other hand, the plasmid pTK-7.5pN constructed in Example 15 was digested with SmaI in a SmaI buffer to form a linearized plasmid DNA. The DNA fragment containing RVF-3 and the linearized plasmid DNA were ligated using a T4 DNA ligase, to contain the RVF-3 downstream of the 7.5K promoter, and the ligation mixture was used to transform E. coli. Plasmids were recovered from the transformants and subjected to restriction enzyme analysis, to select a plasmid wherein RVF-3 has been inserted downstream of and in the same orientation as the 7.5K promoter. This plasmid was designated pTK-7.5pN-RVF-3.

### Example 18 Construction of recombinant virus by transfection of vaccinia virus mO with pHA-7.5pN-RVH or pHA-ATIp-RVH

An Eagle's minimum essential medium containing 5% of fetal bovine serum in a 25 cm bottle was inoculated with a rabbit renal cell line RK13, and culturing was carried out at 37°C in 5% CO₂ to form a monolayer. Next, the vaccinia virus mO as a vector was absorbed to the cultured cells at 0.1 m.o.i for 1 hour at 37°C. Next, the plasmid pHA-7.5pN-RVH constructed in Example 3, or the plasmid pHA-ATIp-RVH constructed in Example 9, was transfected to the cells using a calcium phosphate method (Weir, J.P. et al. Proc Natl. Acad. Si. USA. 79, 1210-1214, 1982). The virus was recovered from the culture by freezing and thawing. RK13 cells were cultured in a 9 cm petri dish to form a monolayer, to which the recovered virus was inoculated at a concentration of 500 to 1000 plaques/petri dish, and culturing was carried out for two days to form plaques. Next, chicken erythrocytes were added to the petri dish to allow absorption of the erythrocytes by the plaques, and plaques which were HA⁻, and therefore do not absorb the erythrocytes, were picked up.

Viruses from the plaques were infected to a monolayer, of RK13 cells in a 3 cm petri dish to allow plaque formation. Next, plaques were transferred to a nylon membrane (Amersham Hybond N), and the membrane was treated with 0.5N NaOH for 5 minutes to denaturate DNA, neutralized by 1M Tris-HCl (pH7.4), and the DNA was adsorbed to the membrane with 1.5M NaCl and 0.5M Tris-HCl (pH7.4), and fixed by ultraviolet radiation (305 nm) for 5 minutes.

To prepare a probe, a plasmid pDH-RVH-2 was digested with BamHI in a high salt concentration buffer, and the digestion product was separated by agarose gel electrophoresis to obtain a DNA fragment containing RVH-2. The DNA fragment was labeled with ³p using a nick-translation kit (Takara).

The probe was hybridized with the above-prepared virus DNA fixed on the membrane at 60°C overnight, and hybridized plaques were detected by autoradiography. As a result, 6 recombinant viruses derived from a recombination with the plasmid pHA-ATIp-RVH, and 6 recombinant viruses derived from a recombination with the plasmid pHA-7.5pN-RVH were obtained and designated mO-ATIp-RVH and mO-7.5pH-RVH respectively.

Moreover, using the same procedure as described above, the above-mentioned plasmids, for example, pHA-ATIp-RVH-3, pHA-7.5pN-RVH-3, pTK-7.5pN-RVF-3, and the like, can be used to construct recombinant vaccinia viruses for use in vaccination against the rinderpest virus infection in an animal.

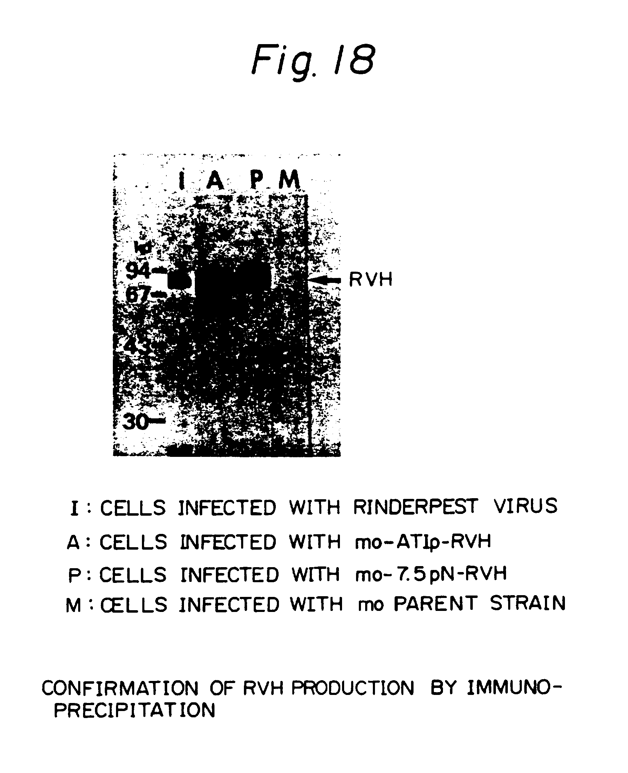

### Example 19 Expression of RVH by recombinant virus (Fig. 18)

RK13 cells were cultured in an Eagle's minimum essential medium containing 10% bovine serum, in a 6 cm petri dish overnight, to form a monolayer. The recombinant virus mO-ATIp-RVH or mO-7.5pN-RVH was infected to the monolayer, and the infected cells were cultured in a modified Eagles minimum essential medium containing 10 mM fructose instead of glucose and containing 100 µci ³H-glucosamine, for 16 hours. The supernatant was subjected to immunoprecipitation using an anti-rinderpest virus rabbit serum and protein A sepharose, to precipitate the rinderpest virus hemagglutinin, and analyzed by SDS-polyacrylamide gel electrophoresis (SDS-PAGE). As a result, as shown in Fig. 18, cells infected with the present recombinant virus produced a glycoprotein having the same mobility as that of a product produced by cells infected with the rinderpest virus, revealing that the rinderpest virus hemagglutinin was expressed by the present recombinant vaccinia virus. A negative control mO strain did not show a band corresponding to that of the positive control product and the expression product of the present recombinant virus.

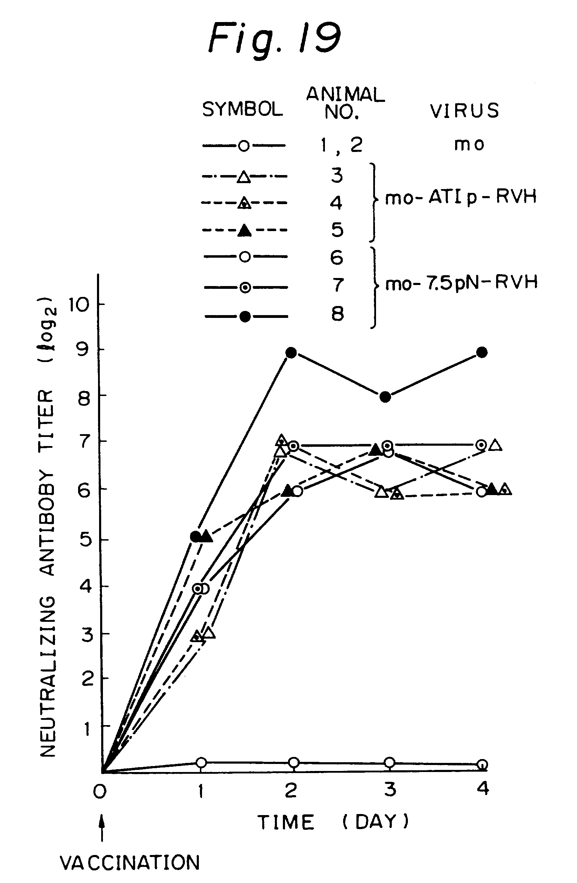

### Example 20 Vaccination of rabbit with recombinant virus and change of rinderpest virus neutralizing antibody titer (Fig. 19)

Three rabbits were vaccinated with the recombinant vaccinia virus mO-7.5pN-RVH, three rabbits with mO-ATIp-RVH, and two rabbits with the parent virus LC16 mO, by subcutaneous injection at 1 x 10⁸ PFU in all cases. A neutralizing antibody titer in serum from each test animal was determined by measuring the neutralizing activity of the serum sample against a vero cell-adapted strain of the rinderpest virus L. As a result, in rabbits vaccinated with mO-7.5pN-RVH and rabbits vaccinated with mO-ATIp-RVH, the neutralizing antibody titer reached 2 to 2⁵ one week after, and a plateau of 2⁶ to 2⁷ two weeks after vaccination. Conversely, the neutralizing antibody activity was not detected in a serum from rabbits vaccinated with the parent virus LC16mO.

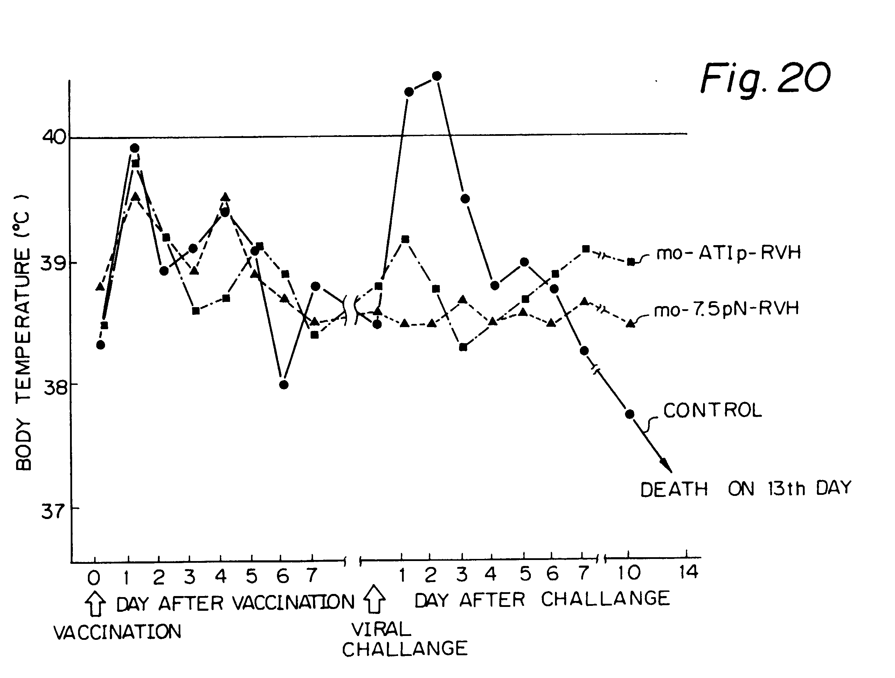

### Example 21 Rinderpest virus infection of rabbit vaccinated with recombinant virus (Fig. 20)

Rinderpest virus R was intravenously infected in an amount of 1 x 10³ ID₅₀ to rabbits vaccinated with the recombinant virus described in Example 21. As seen from Fig. 20, although immediately after vaccination the body temperature was increased, and gradually returned to a normal level in rabbits vaccinated with mO-ATIp-RVH, in rabbit vaccinated with mO-7.5pN-RVH and in rabbits vaccinated with Lc16mo, after the infection with the rinderpest virus, the body temperature was increased only in rabbits vaccinated with Lo16mO, and maintained at a normal level in rabbits vaccinated with mO-ATIp-RVH and rabbits vaccinated with mO-7.5pN-RVH. Further, the rabbits vaccinated with Lc16mO and infected with the rinderpest virus exhibited diarrhea, decrease or loss of appetite, decrease of lymphocytes, and increase of neutrophils three days after the viral infection and died on the 13th day after the viral infection. On the contrary, rabbits vaccinated with the present recombinant virus mO-7.5pH-RVH or with mO-ATIp-RVH prior to the viral infection, exhibited a normal appetite, normal body weight, and normal levels of lymphocytes and neutraphils.

### Example 22 Detection of anti-rinderpest virus antibody in rabbit vaccinated with recombinant virus

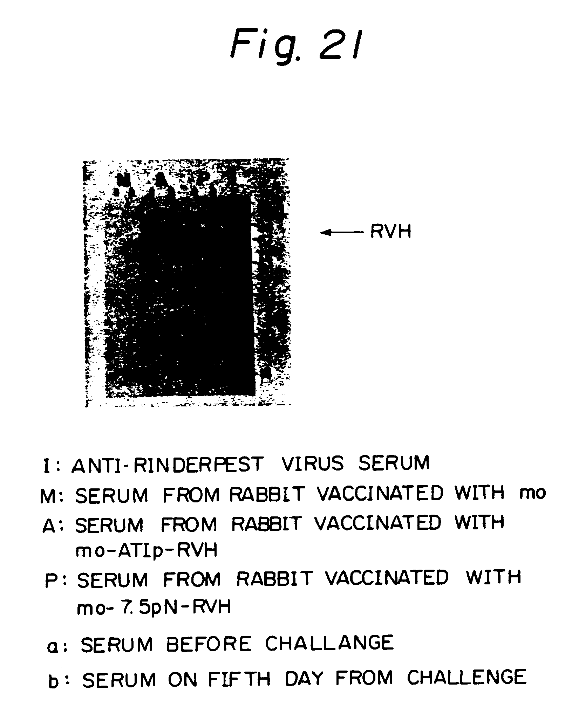

Rabbits were subcutaneously vaccinated with the present recombinant virus mO-ATIp-RVH or mO-7.5pN-RVH, or a control vaccinia virus Lc16mO, and were infected with the rinderpest virus four weeks after the vaccination. Blood samples were obtained prior to the viral infection, and five days after the viral infection. Serum samples from the blood samples were tested for the presence of the anti-rinderpest virus antibody by immunoprecipitation, using ³⁵S-methioninelabeled rinderpest virus-infected cells as an antigen. The results are shown in Fig. 21, which includes the results from the anti-rinderpest virus serum. Induction of anti-rinderpest virus antibody prior to the viral infection with rinderpest virus means that the present recombinant vaccinia virus per se, which was subcutaneously grown, can induce the production of an anti-rinderpest virus antibody without causing a rinderpest virus infection.

### Example 23 Construction of recombinant virus by transfection of vaccinia virus mO with pHA-7.5pN-RVH-3

An Eagle's minimum essential medium containing 5% of fetal bovine serum in a 25 cm bottle was inoculated with a rabbit renal cell line RK13, and culturing was carried out at 37°C in 5% CO₂ to form a monolayer. Next, the vaccinia virus mO as a vector was absorbed to the cultured cells at 0.1 m.o.i for 1 hour at 37°C. Then, the plasmid pHA-7.5pN-RVH-3 constructed in Example 13, was transfected to the cells using a calcium phosphate method (Weir, J.P. et al. Proc Natl. Acad. Si. USA. 79, 1210-1214, 1982). The virus was recovered from the culture by freezing and thawing. RK13 cells were cultured in a 9 cm petri dish to form a monolayer, to which the recovered virus was inoculated at a concentration of 500 to 1000 plaques/petri dish, and culturing was carried out for two days to form plaques. Next, chicken erythrocytes were added to the petri dish to allow absorption of the erythrocytes by the plaques, and plaques which were HA⁻, and therefore do not absorb the erythrocytes, were picked up.

Viruses from the plaques were infected to a monolayer, of RK13 cells in a 3 cm petri dish to allow plaque formation. Next, plaques were transferred to a nylon membrane (Amersham Hybond N), and the membrane was treated with 0.5N NaOH for 5 minutes to denaturate DNA, neutralized by 1M Tris-HCl (pH7.4), and the DNA was adsorbed to the membrane with 1.5M NaCl and 0.5M Tris-HCl (pH7.4), and fixed by ultraviolet radiation (305 nm) for 5 minutes.

To prepare a probe, a plasmid pDH-RVH-2 was digested with BamHI in a high salt concentration buffer, and the digestion product was separated by agarose gel electrophoresis to obtain a DNA fragment containing RVH-2. The DNA fragment was labeled with ³p using a nick-translation kit (Takara).

The probe was hybridized with the above-prepared virus DNA fixed on the membrane at 60°C overnight, and hybridized plaques were detected by autoradiography. As a result, one recombinant viruses derived from a recombination with the plasmid pHA-7.5pN-RVH-3 was obtained and designated mO-7.5pH-RVH-3.

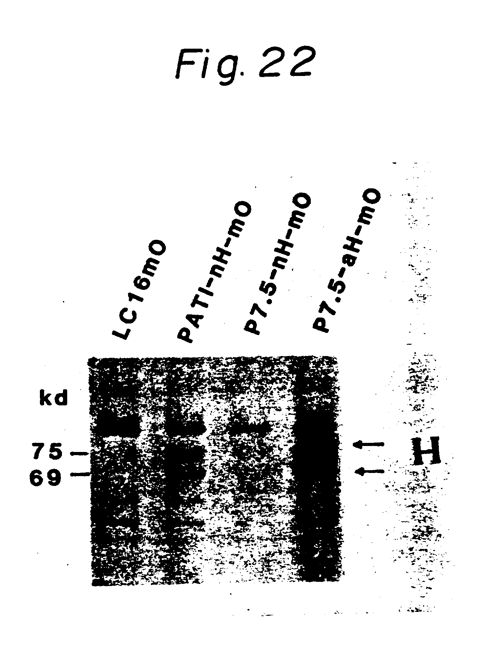

### Example 24 Expression of RVH by recombinant virus (Fig. 22)

RK13 cells were cultured in an Eagle's minimum essential medium containing 10% bovine serum, in a 6 cm petri dish overnight, to form a monolayer. The recombinant virus mO-7.5pN-RVH-3, mO-ATIp-RVH or mO-7.5pN-RVH was infected to the monolayer, and the infected cells were cultured in an EMEM medium containing 50 µci ³⁵S-methionine, for 16 hours. The supernatant was subjected to immunoprecipitation using an anti-measles virus hemagglutinin rabbit serum and protein A sepharose, to precipitate the rinderpest virus hemagglutinin, and separated by SDS-polyacrylamide gel electrophoresis (SDS-PAGE), and analyzed by autoradiography. As a result, as shown in Fig. 22, cells infected with the present recombinant virus produced a glycoprotein of about 75Kd having the same mobility as that of a product produced by cells infected with the rinderpest virus, as well as a protein of about 69Kd considered to be a precursor of the 75 Kd protein, revealing that the rinderpest virus hemagglutinin was expressed by the present recombinant vaccinia virus. A negative control mO strain did not show a band corresponding to that of the positive control product and the expression product of the present recombinant virus.

The densities of the bands were compared by densitometry, and as a result, it was estimated that the recombinant virus mO-7.5pN-RVH-3 produced the rinderpest virus hemagglutinin in an amount of as much as ten times that of mO-7.5pN-RVH.

Note in Fig. 22, "LC16mo" corresponds to the control mO strain; "PAT1-nH-mO" corresponds to the strain mO-ATIp-RVH; "P7.5-nH-mO" corresponds to mO-7.5pN-RVH; and "P7.5-aH-mO" corresponds to mO-7.5pN-RVH-3.

## Claims

1. A plasmid comprising a gene coding for an antigenic protein of a rinderpest virus and a viral promoter which can express said gene, as well as a gene derived from a vaccinia virus which is not essential for the growth of the vaccinia virus, wherein the gene coding for the antigenic protein and the viral promoter have been inserted in the gene not essential for the growth of the vaccinia virus;
characterized in that:
(1) the viral promoter is vaccinia virus 7.5 K protein gene promoter (7.5 Kp) and the vaccinia virus gene not essential for the growth of vaccinia virus is a vaccinia virus hemagglutinin gene (H); or
(2) the viral promoter is poxvirus A type inclusion body gene promoter (ATIp), and the vaccinia virus gene not essential for the growth of vaccinia virus is H; or
(3) the viral promoter is ATIp, and the vaccinia virus gene not essential for the growth of vaccinia virus is vaccinia virus thymidine kinase gene (TK).

2. A recombinant vaccinia virus LC16m0 or LC16m8, wherein a gene coding for an antigenic protein of a rinderpest virus and a viral promoter which can express said gene have been inserted in a gene which is not essential for the growth of the vaccinia virus LC16m0 or LC16m8.

3. A recombinant vaccinia virus according to claim 2 wherein the gene coding for the antigenic protein of a rinderpest virus is either a gene coding for rinderpest virus hemagglutinin protein (RVH) or part thereof or a gene coding for rinderpest virus fusion protein (RVF) or part thereof, the viral promoter which can express said gene is either a promoter of a poxvirus A type inclusion body gene (ATIp) or a promoter of a vaccinia virus 7.5 K protein gene (7.5 Kp) and the gene not essential for the growth of vaccinia virus is either vaccinia virus hemagglutinin gene (H) or vaccinia virus thyridine kinase gene (TK).

4. A recombinant vaccinia virus according to claim 3, wherein the RVH and the ATIp have been inserted in the H gene of the vaccinia virus.

5. A recombinant vaccinia virus according to claim 3, wherein the RVH and the 7.5 Kp have been inserted in the H gene of the vaccinia virus.

6. A recombinant vaccinia virus according to claim 3, wherein the RVF and the 7.5 Kp have been inserted in the TK gene of the vaccinia virus.

7. A recombinant vaccinia virus according to claim 3, wherein the RVH and the ATIp have been inserted in the TK gene of the vaccinia virus.

8. A recombinant vaccinia virus according to claim 3, wherein the RVH and the 7.5 Kp have been inserted in the TK gene of the vaccinia virus.

9. A recombinant vaccinia virus according to claim 3, wherein the RVF and the ATIp have been inserted in the H gene of the vaccinia virus.

10. A recombinant vaccinia virus according to claim 3, wherein the RVF and the 7.5 Kp have been inserted in the H gene of the vaccinia virus.

11. A recombinant vaccinia virus according to claim 3, wherein the RVF and the ATIp have been inserted in the TK gene of the vaccinia virus.

12. A process for a production of a recombinant vaccinia virus comprising the steps of,
(1) preparing a plasmid comprising a gene coding for an antigenic protein of the rinderpest virus and a viral promoter which can express said gene, as well as a gene derived from the vaccinia virus which is not essential for the growth of the vaccinia virus, wherein the gene coding for the antigenic protein and the viral promoter have been inserted in the gene not essential for the growth of the vaccinia virus LC16m0 or LC16m8.
(2) subjecting the plasmid to recombination with vaccinia virus LC16m0 or LC16m8; and
(3) selecting a recombinant vaccinia virus wherein a gene corresponding to the gene not essential for the growth of the vaccinia virus has been inactivated.

13. A recombinant vaccinia virus prepared by the process according to claim 12.

14. A vaccine against the rinderpest virus comprising, a recombinant vaccina virus LC16m0 or LC16m8, wherein a gene coding for an antigenic protein of the rinderpest virus and a viral promoter which can express said gene have been inserted in a gene which is not essential for the growth of vaccinia virus LC16m0 or LC16m8.

## Patentansprüche

1. Ein Plasmid, welches ein Gen, das für ein antigenes Protein eines Rinderpestvirus codiert, und einen viralen Promotor, der das Gen exprimieren kann, wie auch ein Gen, das von einem Vacciniavirus abgeleitet ist, welches nicht essentiell für das Wachstum des Vacciniavirus ist, umfaßt, wobei das Gen, welches für das antigene Protein codiert, und der virale Promotor in das Gen eingefügt wurden, welches nicht essentiell für das Wachstum des Vacciniavirus ist;
dadurch gekennzeichnet, daß:
(1) der virale Promotor Vacciniavirus-7,5 K-Proteingen-Promotor (7,5 Kp) ist und das Vacciniavirusgen, welches nicht essentiell für das Wachstum von Vacciniavirus ist, ein Vacciniavirus-Hämagglutiningen (H) ist; oder
(2) der virale Promotor Pockenvirus-A-Typ-Einschlußkörper-Genpromotor (ATIp) ist und das Vacciniavirusgen, welches nicht essentiell für das Wachstum von Vacciniavirus ist, H ist; oder
(3) der virale Promotor ATIp ist und das Vacciniavirusgen, welches nicht essentiell für das Wachstum von Vacciniavirus ist, Thymidinkinasegen (TK) ist.

2. Ein rekombinantes Vacciniavirus LC16m0 oder LC16m8, wobei ein Gen, das für ein antigenes Protein eines Rinderpestvirus codiert, und ein viraler Promotor, der das Gen exprimieren kann, in ein Gen eingefügt wurden, welches nicht essentiell für das Wachstum des Vacciniavirus LC16m0 oder LC16m8 ist.

3. Ein rekombinantes Vacciniavirus gemäß Anspruch 2, wobei das Gen, welches für das antigene Protein eines Rinderpestvirus codiert, entweder ein Gen ist, das für Rinderpestvirus-Hämagglutininprotein (RVH) oder einen Teil davon codiert, oder ein Gen ist, das für Rinderpestvirus-Fusionsprotein (RVF) oder einen Teil davon codiert, der virale Promotor, der das Gen exprimieren kann, entweder ein Promotor eines Pockenvirus-A-Typ-Einschlußkörpergens (ATIp) oder ein Promotor eines Vacciniavirus-7,5 K-Proteingens (7,5 Kp) ist und das Gen, welches nicht essentiell für das Wachstum von Vacciniavirus ist, entweder Vacciniavirus-Hämagglutiningen (H) oder Vacciniavirus-Thymidinkinasegen (TK) ist.

4. Ein rekombinantes Vacciniavirus gemäß Anspruch 3, wobei das RVH und der ATIp in das H-Gen des Vacciniavirus eingefügt wurden.

5. Ein rekombinantes Vacciniavirus gemäß Anspruch 3, wobei das RVH und der 7,5 Kp in das H-Gen des Vacciniavirus eingefügt wurden.

6. Ein rekombinantes Vacciniavirus gemäß Anspruch 3, wobei das RVF und der 7,5 Kp in das TK-Gen des Vacciniavirus eingefügt wurden.

7. Ein rekombinantes Vacciniavirus gemäß Anspruch 3, wobei das RVH und der ATIp in das TK-Gen des Vacciniavirus eingefügt wurden.

8. Ein rekombinantes Vacciniavirus gemäß Anspruch 3, wobei das RVH und der 7,5 Kp in das TK-Gen des Vacciniavirus eingefügt wurden.

9. Ein rekombinantes Vacciniavirus gemäß Anspruch 3, wobei das RVF und der ATIp in das H-Gen des Vacciniavirus eingefügt wurden.

10. Ein rekombinantes Vacciniavirus gemäß Anspruch 3, wobei das RVF und der 7,5 Kp in das H-Gen des Vacciniavirus eingefügt wurden.

11. Ein rekombinantes Vacciniavirus gemäß Anspruch 3, wobei das RVF und der ATIp in das TK-Gen des Vacciniavirus eingefügt wurden.

12. Ein Verfahren zur Herstellung eines rekombinanten Vacciniavirus, welches die folgenden Schritte umfaßt:
(1) Herstellen eines Plasmides, welches ein Gen, das für ein antigenes Protein des Rinderpestvirus codiert, und einen viralen Promotor, der das Gen exprimieren kann, wie auch ein Gen, das von dem Vacciniavirus abgeleitet ist, welches nicht essentiell für das Wachstum des Vacciniavirus ist, umfaßt, wobei das Gen, das für das antigene Protein codiert, und der virale Promotor in das Gen, welches nicht essentiell für das Wachstum des Vacciniavirus LC16m0 oder LC16m8 ist, eingefügt wurden;
(2) Unterwerfen des Plasmides unter eine Rekombination mit Vacciniavirus LC16m0 oder LC16m8; und
(3) Selektieren eines rekombinanten Vacciniavirus, wobei ein Gen, welches dem Gen entspricht, das nicht essentiell für das Wachstum des Vacciniavirus ist, inaktiviert wurde.

13. Ein rekombinantes Vacciniavirus, welches durch das Verfahren gemäß Anspruch 12 hergestellt wurde.

14. Ein Impfstoff gegen das Rinderpestvirus, welcher ein rekombinantes Vacciniavirus LC16m0 oder LC16m8 umfaßt, wobei ein Gen, das für ein antigenes Protein des Rinderpestvirus codiert, und ein viraler Promotor, der das Gen exprimieren kann, in ein Gen eingefügt wurden, das nicht essentiell für das Wachstum von Vacciniavirus LC16m0 oder LC16m8 ist.

## Revendications

1. Plasmide, comprenant une codification de gène d'une protéine antigène de virus de la peste bovine et un promoteur viral pouvant exprimer ledit gène, ainsi qu'un gène dérivé d'un virus de la vaccine qui n'est pas essentiel pour la croissance du virus de la vaccine, dans lequel la codification de gène de la protéine antigène et le promoteur viral ont été insérés dans le gène non essentiel pour la croissance du virus de la vaccine,
caractérisé en ce que:
(1) le promoteur viral est le promoteur de gène de protéine 7.5 Kpde virus de la vaccine (7.5 K) et le gène de virus de la vaccine non essentiel pour la croissance du virus de la vaccine est un gène d'hémagglutinine de virus de la vaccine (H), ou
(2) le promoteur viral est le promoteur de gène de corps d'inclusion du type virus de la vaccine A (ATIp), et le gène de virus de la vaccine non essentiel pour la croissance du virus de la vaccine est H, ou
(3) le promoteur viral est l'ATIp, et le gène de virus de la vaccine non essentiel pour la croissance du virus de la vaccine est un gène de kinase de thymidine de virus de la vaccine (TK).

2. Virus de la vaccine recombinant LC16m0 ou LC16m8, dans lequel une codification de gène d'une protéine antigène de virus de la peste bovine et un promoteur viral pouvant exprimer ledit gène ont été insérés dans un gène qui n'est pas essentiel pour la croissance du virus de la vaccine LC16m0 ou LC16m8.

3. Virus de la vaccine recombinant suivant la revendication 2, dans lequel la protéine antigène de virus de la peste bovine est soit une codification de gène de protéine d'hémagglutinine de virus de la vaccine (RVH), ou une partie de celle-ci, soit une codification de gène de protéine de fusion de virus de la vaccine (RVF), ou une partie de celle-ci, le promoteur viral qui peut exprimer ledit gène est soit un promoteur d'un gène de corps d'inclusion du type virus de la vaccine A (ATIp) ou un promoteur de gène de protéine 7.5 K de virus de la vaccine (7.5 K) et le gène non essentiel pour la croissance du virus de la vaccine est soit un gène d'hémagglutinine de virus de la vaccine (H), soit un gène de kinase de thymidine de virus de la vaccine (TK).

4. Virus de la vaccine recombinant suivant la revendication 3, dans lequel la RVH et l'ATIp ont été insérés dans le gène H du virus de la vaccine.

5. Virus de la vaccine recombinant suivant la revendication 3, dans lequel la RVH et le 7.5 K ont été insérés dans le gène H du virus de la vaccine.

6. Virus de la vaccine recombinant suivant la revendication 3, dans lequel la RVF et le 7.5 K ont été insérés dans le gène TK du virus de la vaccine.

7. Virus de la vaccine recombinant suivant la revendication 3, dans lequel la RVH et l'ATIp ont été insérés dans le gène TK du virus de la vaccine.

8. Virus de la vaccine recombinant suivant la revendication 3, dans lequel la RVH et le 7.5 K ont été insérés dans le gène TK du virus de la vaccine.

9. Virus de la vaccine recombinant suivant la revendication 3, dans lequel la RVF et l'ATIp ont été insérés dans le gène H du virus de la vaccine.

10. Virus de la vaccine recombinant suivant la revendication 3, dans lequel la RVF et le 7.5 K ont été insérés dans le gène H du virus de la vaccine.

11. Virus de la vaccine recombinant suivant la revendication 3, dans lequel la RVF et l'ATIp ont été insérés dans le gène TK du virus de la vaccine.

12. Procédé de production d'un virus de la vaccine recombinant, comprenant les étapes consistant à
(1) préparer un plasmide, comprenant une codification de gène d'une protéine antigène de virus de la peste bovine et un promoteur viral pouvant exprimer ledit gène, ainsi qu'un gène dérivé d'un virus de la vaccine qui n'est pas essentiel pour la croissance du virus de la vaccine, dans lequel la codification de gène de la protéine antigène et le promoteur viral ont été insérés dans le gène qui n'est pas essentiel pour la croissance du virus de la vaccine LC16m0 ou LC16m8,
(2) soumettre le plasmide à recombinaison avec le virus de la vaccine LC16m0 ou LC16m8, et
(3) choisir un virus de la vaccine recombinant dans lequel a été inactivé un gène correspondant au gène non essentiel pour la croissance du virus de la vaccine.

13. Virus de la vaccine recombinant préparé par le procédé suivant la revendication 12.

14. Vaccin contre le virus de la peste bovine, comprenant un virus de la vaccine recombinant LC16m0 ou LC16m8, dans lequel une codification de gène d'une protéine antigène de virus de la peste bovine et un promoteur viral pouvant exprimer ledit gène ont été insérés dans un gène qui n'est pas essentiel pour la croissance du virus de la vaccine LC16m0 ou LC16m8.
